# EUROPEAN PATENT SPECIFICATION

(11) **EP 4 430 618 B1**
(45) Date of publication and mention of the grant of the patent: **06.08.2025**
(21) Application number: 22813495.3
(22) Date of filing: 02.11.2022
(51) Int. Cl.: G16C 20/70, G16C 20/30, G16C 60/00

(54) **AUTOENCODING FORMULATIONS**
AUTOCODIERUNGSFORMULIERUNGEN
FORMULATIONS À CODAGE AUTOMATIQUE

(30) Priority: 08.11.2021 EP 21206842
(43) Date of publication of application: 18.09.2024
(73) Proprietor: Bayer Aktiengesellschaft, 51373 Leverkusen (DE)
(72) Inventor: HAESE, Florian, 51373 Leverkusen (DE); HATZ, Kathrin, 51373 Leverkusen (DE); KLEMMER, Helge Frederic Maria, 51373 Leverkusen (DE); WEISS, Martin, 51373 Leverkusen (DE); GRIMBS, Sergio, 51373 Leverkusen (DE); KAUSCH-BUSIES, Nina, 51373 Leverkusen (DE); NIEDENFUEHR, Sebastian, 51373 Leverkusen (DE)
(74) Representative: BIP Patents
(86) International application number: PCT/EP2022/080530
(87) International publication number: WO 2023/078914

(56) References cited:
- WO-A1-2021/056116
- WO-A1-2021/165386
- US-A1- 2006 031 027
- SEVGEN E. ET AL: "Toward Predictive Chemical Deformulation Enabled by Deep Generative Neural Networks", INDUSTRIAL & ENGINEERING CHEMISTRY RESEARCH, vol. 60, no. 39, 23 September 2021 (2021-09-23), pages 14176 - 14184, XP093222488, ISSN: 0888-5885, Retrieved from the Internet <URL:https://pubs.acs.org/doi/pdf/10.1021/acs.iecr.1c00634> DOI: 10.1021/acs.iecr.1c00634
- BENNETT-LENANE HARRIET ET AL: "Artificial Neural Networks to Predict the Apparent Degree of Supersaturation in Supersaturated Lipid-Based Formulations: A Pilot Study", PHARMACEUTICS, vol. 13, no. 9, 5 September 2021 (2021-09-05), pages 1398, XP055916246, DOI: 10.3390/pharmaceutics13091398

## Description

### FIELD

Systems, methods, and computer programs disclosed herein relate to training and using a machine learning model to predict compositions and/or properties of formulations, in particular of active ingredient formulations.

### BACKGROUND

WO2021/165386 A1 discloses a method, a computer system, and a computer program product for predicting at least one property of at least one formulation using a prediction model which has been trained to predict formulation properties by means of a monitored learning process using reference data. WO2021/056116A1 discloses a computer system that predicts synergistic interactions between pesticidal and synergistic compounds of a pesticidal composition.

H. Bennet-Lenane *et al.* disclose artificial neural networks to predict the apparent degree of supersaturation in supersaturated lipid-based formulations (H. Bennet-Lenane et al.: Artificial Neural Networks to Predict the Apparent Degree of Supersaturation in Supersaturated Lipid-Based Formulations: A Pilot Study, Pharmaceutics 2021, 13, 1398).

US2006/031027A1 discloses a method and apparatus for predicting the non-color properties of a chemical mixture, such as an automotive paint, using an artificial neural network.

A formulation is a mixture of different substances. A formulation is produced from defined quantities of the substances according to a recipe. A formulation is usually used to bring one or more ingredients of the formulation into a form that fulfills a defined purpose. In the case of pharmaceuticals, crop protection products, and pesticides (such as insecticides, fungicides, and/or herbicides), one or more active ingredients are usually brought into a form with formulation aids that improves the biological effect of the active ingredient(s) in a target organism compared with the pure active ingredient(s) and/or makes the finished product applicable for a defined purpose (e.g., application of a pesticide by means of a particular application technology) and/or provides chemical stability of the active ingredient(s) under defined conditions

In the development of a formulation, usually different formulations are generated, some of their properties tested and compared to identify a formulation suitable for a defined purpose. Usually, the goal is to identify an "optimized" formulation for the intended application, often involving optimization with respect to various target parameters (such as environmental compatibility, bioavailability, manageability, costs etc.). Formulation development usually strives to optimize multiple properties at once (or at least generate formulations of which pre-defined thresholds for multiple properties are satisfied simultaneously).

To guide and/or reduce the experimental effort in developing a new formulation, it would be desirable to have decision support tools available for the selection of possible formulations and/or for predicting their properties, particularly for predicting the effect one or more ingredients added to or removed from a formulation on one or more properties of the formulation.

### SUMMARY OF THE INVENTION

The invention is defined in the appended claims.

### SUMMARY OF RELATED DISCLOSURE

The present disclosure provides a solution for identifying promising formulation candidates and/or predicting properties of formulation candidates.

In a first aspect, the present disclosure provides a computer-implemented method for training a machine learning model, the method comprising:
- receiving information about a multitude of formulations, the information comprising composition information about compositions of formulations, and property information about one or more properties of formulations,
- generating training data from the information about the multitude of formulations, the training data comprising composition data, and property data;
- providing a machine learning model;
- training the machine learning model, thereby generating a trained machine learning model, wherein the training comprises:
   ∘ inputting composition data into the machine learning model, wherein the machine learning model is configured to
      ▪ generate a compressed representation of the formulation, at least partially on the basis of the composition data,
      ▪ determine predicted property data, at least partially on the basis of the compressed representation of the formulation,
      ▪ generate reconstructed composition data, at least partially on the basis of the compressed representation of the formulation,
   ∘ computing a loss by means of one or more loss function(s), the loss function(s) quantifying deviations between at last a part of the composition data and the reconstructed composition data, and between at least a part of the property data and the predicted property data,
   ∘ modifying parameters of the machine learning model so that the loss is minimized,
- storing the trained machine learning model and/or outputting the trained machine learning model and/or using the trained machine learning model for prediction purposes.

In another aspect, the present disclosure provides a computer system comprising:
a processor; and
a memory storing an application program configured to perform, when executed by the processor, an operation, the operation comprising:
   - receiving information about a multitude of formulations, the information comprising composition information about compositions of formulations, and property information about one or more properties of formulations,
   - generating training data from the information about the multitude of formulations, the training data comprising composition data, and property data;
   - providing a machine learning model;
   - training the machine learning model, thereby generating a trained machine learning model, wherein the training comprises:
      o inputting composition data into the machine learning model, wherein the machine learning model is configured to
         ▪ generate a compressed representation of the formulation, at least partially on the basis of the composition data,
         ▪ determine predicted property data, at least partially on the basis of the compressed representation of the formulation,
         ▪ generate reconstructed composition data, at least partially on the basis of the compressed representation of the formulation,
      ∘ computing a loss by means of one or more loss function(s), the loss function(s) quantifying deviations between at last a part of the composition data and the reconstructed composition data, and between at least a part of the property data and the predicted property data,
      ∘ modifying parameters of the machine learning model so that the loss is minimized,
   - storing the trained machine learning model and/or outputting the trained machine learning model and/or using the trained machine learning model for prediction purposes.

In another aspect, the present disclosure provides a non-transitory computer-readable storage medium having stored thereon software instructions that, when executed by a processor of a computer system, cause the computer system to execute the following steps:
- receiving information about a multitude of formulations, the information comprising composition information about compositions of formulations, and property information about one or more properties of formulations,
- generating training data from the information about the multitude of formulations, the training data comprising composition data, and property data;
- providing a machine learning model;
- training the machine learning model, thereby generating a trained machine learning model, wherein the training comprises:
   ∘ inputting composition data into the machine learning model, wherein the machine learning model is configured to
      ▪ generate a compressed representation of the formulation, at least partially on the basis of the composition data,
      ▪ determine predicted property data, at least partially on the basis of the compressed representation of the formulation,
      ▪ generate reconstructed composition data, at least partially on the basis of the compressed representation of the formulation,
   ∘ computing a loss by means of one or more loss function(s), the loss function(s) quantifying deviations between at last a part of the composition data and the reconstructed composition data, and between at least a part of the property data and the predicted property data,
   ∘ modifying parameters of the machine learning model so that the loss is minimized,
- storing the trained machine learning model and/or outputting the trained machine learning model and/or using the trained machine learning model for prediction purposes.

Further aspects are disclosed in the independent claims, the specification, and the drawings.

### DETAILED DESCRIPTION

The invention will be more particularly elucidated below without distinguishing between the aspects of the invention (method, computer system, computer-readable storage medium). On the contrary, the following elucidations are intended to apply analogously to all the aspects of the invention, irrespective of in which context (method, computer system, computer-readable storage medium) they occur.

If steps are stated in an order in the present description or in the claims, this does not necessarily mean that the invention is restricted to the stated order. On the contrary, it is conceivable that the steps can also be executed in a different order or else in parallel to one another, unless one step builds upon another step, this absolutely requiring that the building step be executed subsequently (this being, however, clear in the individual case). The stated orders are thus preferred embodiments of the present disclosure.

As used herein, the articles "a" and "an" are intended to include one or more items and may be used interchangeably with "one or more" and "at least one." As used in the specification and the claims, the singular form of "a", "an", and "the" include plural referents, unless the context clearly dictates otherwise. Where only one item is intended, the term "one" or similar language is used. Also, as used herein, the terms "has", "have", "having", or the like are intended to be open-ended terms. Further, the phrase "based on" is intended to mean "based at least partially on" unless explicitly stated otherwise.

Some implementations of the present disclosure will be described more fully hereinafter with reference to the accompanying drawings, in which some, but not all implementations of the disclosure are shown. Indeed, various implementations of the disclosure may be embodied in many different forms and should not be construed as limited to the implementations set forth herein; rather, these example implementations are provided so that this disclosure will be thorough and complete, and will fully convey the scope of the disclosure to those skilled in the art.

The present disclosure provides means for identification of promising formulation candidates and/or for the prediction of formulation properties.

The term "formulation", as used herein, preferably refers to a mixture of ingredients (e.g., substances) prepared according to a specific (e.g., defined) recipe which can be used for a specific (e.g., defined) purpose.

Preferably, a formulation according to the present disclosure comprises at least one biologically active ingredient. Biologically active ingredients, also referred to as active ingredients for short, are substances that have a specific effect or cause a specific reaction in a biological organism. Examples of such biologically active ingredients are drugs (pharmaceutically active substances), crop protection agents (e.g., herbicides, insecticides, fungicides) or (other) pest control agents (e.g., other biocides such as bactericides, nematicides, molluscicides, rodenticides and the like).

A "substance" in the sense of the present disclosure is preferably a chemical compound in the form of a pure substance which has a defined chemical structure. The chemical structure reflects the structure at the molecular (or ionic) level. However, the definition of substance given here should not be understood to mean that a formulation in the sense of this disclosure is built only from structurally known components. It may, for example, also include ingredients from biological sources whose chemical structure may be non-uniform or even unknown, or which may consist of a large number of individual components that may vary and/or some of which may still be unnamed.

Preferably, the active ingredient is an organic compound. An "organic compound" is a chemical compound comprising carbon-hydrogen bonds (C-H bonds). Preferably, the active ingredient is an organic compound whose molecules are composed of only the following elements: Carbon (C), Hydrogen (H), Oxygen (O), Nitrogen (N), Sulfur (S), Fluorine (F), Chlorine (Cl), Bromine (Br), Iodine (I) and/or Phosphorus (P).

Typically, the active ingredient is a solid or a liquid under standard conditions (temperature: 273.15 K, pressure: 1 bar).

In a formulation, one or more active ingredients can be mixed with one or more other substances.

When a formulation is created, the active ingredient(s) to be formulated is/are put into a form that is particularly useful for its planned application (intended use). In the case of a formulation, therefore, the intended application (the intended use) can be decisive for the properties to be optimized.

Preferably, the formulation is an aqueous formulation, i.e., a formulation containing water.

In an embodiment of the present disclosure, the formulation is a (e.g., pharmaceutical) active ingredient formulation which aims to increase the solubility in a defined medium and/or the bioavailability of the active ingredient and/or to adjust the release rate in a defined manner.

In a further embodiment of the present disclosure, the formulation is a phytomedicinal formulation of a pesticide with adjuvants (formulation aids) to enable the application of the formulation by means of a particular application technology (e.g., spray application, rotating disc application, drop application and/or others).

In a further embodiment of the present disclosure, the formulation is a preparation form for a crop protection product and/or pest control agent, comprising one or more active ingredients and formulation adjuvants, in which the biological effect of the active ingredient in the target organism is to be optimized and/or the finished product is to be converted into a form that can be used with a particular equipment.

In a further embodiment of the present disclosure, the formulation is an active ingredient formulation, preferably for crop protection, which is characterized by a pre-defined storage stability and/or viscosity.

The basis of the present disclosure is a machine learning model that is trained to learn correlations within and between compositions of formulations and one or more properties of formulations in order to, e.g., predict the one or more properties of new and/or modified formulations and/or identify new and/or modified formulations with defined properties based on these learned correlations.

Such a "machine learning model", as used herein, may be understood as a computer implemented data processing architecture. The machine learning model can receive input data and provide output data based on that input data and the machine learning model, in particular parameters of the machine learning model. The machine learning model can learn a relation between input data and output data through training. In training, parameters of the machine learning model may be adjusted in order to provide a desired output for a given input.

The process of training a machine learning model involves providing a machine learning algorithm (that is the learning algorithm) with training data to learn from. The term "trained machine learning model" refers to the model artifact that is created by the training process. The training data must contain the correct answer, which is referred to as the target. The learning algorithm finds patterns in the training data that map input data to the target, and it outputs a trained machine learning model that captures these patterns.

The training data are generated from information about a multitude of formulations.

The term "multitude" as it is used herein means an integer greater than 1, usually greater than 10, preferably greater than 100.

The information about the multitude of formulations comprises composition information about the compositions of the formulations, and property information about one or more properties of the formulations. Said information about the multitude of formulations can be obtained (e.g., received) from one or more (e.g., internal and/or external) databases and/or determined experimentally.

It should be noted that composition information and property information need not be available for every formulation. It is for example possible, that there are formulations for which only composition information is available.

Thus, the information about a multitude of formulations comprises, for a number *m* of formulations, (only) composition information related to the composition of each formulation, and for a number *M* of formulations, composition information related to the composition of each formulation and property information related to one or more properties of each formulation, wherein *m* is an integer equal to or greater than 0 and *M* is an integer greater than 1 (preferably greater than 100).

It is also possible that for one or more formulations only part of the property information is available.

For training purposes, the information about a multitude of formulations must be put into a form that can be processed by the machine learning model. So, from the composition information composition data are generated and from the property information property data are generated. The composition data, the property data, and optionally further data constitute the training data. Preferred ways to generate the training data from the information about the multitude of formulations are described in more detail below.

In the training process, composition data (and optionally further data) are inputted into the machine learning model. The machine learning model is configured and trained to perform two tasks, preferably simultaneously, at least partially on the basis of the composition data: a reconstruction task, and a prediction task. The reconstruction task aims at reconstructing some or all composition data. The prediction task aims at predicting some or all property data (associated with the respective composition data). The result of the reconstruction task is herein referred to as reconstructed composition data; the result of the prediction task is herein referred to as predicted property data.

Thus, the machine learning model generates two outputs: reconstructed composition data and predicted property data. The reconstructed composition data can be compared with at least a part of the (original) composition data (first target), and the predicted property data can be compared with at least a part of the (original) property data (second target). Parameters of the machine learning model can be modified in order to reduce the deviations between the two outputs and the respective targets to a (defined) minimum.

In general, a loss function can be used for training to evaluate the machine learning model. For example, a loss function can include a metric of comparison of an output and the respective target. The loss function may be chosen in such a way that it rewards a wanted relation between output and target and/or penalizes an unwanted relation between an output and the respective target. Such a relation can be, e.g., a similarity, or a dissimilarity, or another relation.

A loss function can be used to calculate a loss for a given pair of output and target. The aim of the training process can be to modify (adjust) parameters of the machine learning model in order to reduce the loss to a (defined) minimum.

A loss function may for example quantify the deviation between an output of the machine learning model for a given input and the respective target. If, for example, an output and the respective target are numbers, the loss can be the absolute difference between these numbers. In this case, a high value of the loss function can mean that a parameter of the model needs to undergo a strong change.

In the case of a scalar output, a loss may be a difference metric such as an absolute value of a difference, a squared difference.

In the case of vector-valued outputs, for example, difference metrics between vectors such as the root mean square error, a cosine distance, a norm of the difference vector such as a Euclidean distance, a Chebyshev distance, an Lp-norm of a difference vector, a weighted norm or any other type of difference metric of two vectors can be chosen. These two vectors may for example be the desired output (target) and the actual output.

In the case of higher dimensional outputs, such as two-dimensional, three-dimensional or higher-dimensional outputs, for example an element-wise difference metric may be used. Alternatively or additionally, the output data may be transformed, for example to a one-dimensional vector, before computing a loss.

Preferred loss functions for training the machine learning model according to the present disclosure are given below.

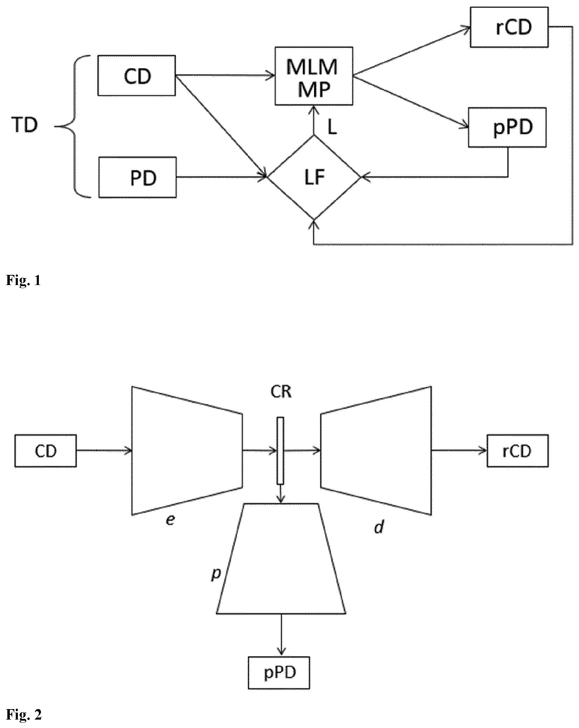

Fig. 1 shows schematically, by way of example, the process of training a machine learning model. The machine learning model MLM is trained on the basis of training data TD. The training data comprise, for a multitude of formulations, i) composition data, and ii) property data.

In the example shown in Fig. 1, only one data set for a single formulation comprising composition data CD and property data PD is shown. The composition data CD is inputted into the machine learning model MLM. The machine learning model is configured to generate, at least partially on the basis of the composition data CD and model parameters MP, reconstructed composition data rCD and predicted property data pPD. The aim of the training can be to reconstruct the whole composition data set from the composition data CD. In this case, the machine learning model MLM is trained to generate reconstructed composition data rCD which come as close as possible to the inputted composition data CD. However, it is also possible to train the machine learning model MLM to only reconstruct one or more parts of the inputted composition data. In this case, the reconstructed composition data rCD represent only a part of the inputted composition data. The same applies to the predicted property data.

The reconstructed composition data rCD can be compared with the composition data CD (or with one or more parts thereof), and the predicted property data pPD can be compared with the property data (or with one or more parts thereof). This can be done by using a loss function LF, the loss function quantifying the deviations between the composition data CD (or one or more parts thereof) and the reconstructed composition data rCD on the one hand side, and the property data PD (or one or more parts thereof) and the predicted property data rPD on the other hand side. For each quartet of composition data, reconstructed composition data, property data, and predicted property data, a loss L can be computed. It is also possible to compute separate losses for each pair of composition data CD and reconstructed composition data rCD, as well as for each pair of property data PD and predicted property data rPD, using separate loss functions for composition data and reconstructed composition data on the one hand side, and for property data and predicted property data on the other hand side.

During training the model parameters MP are modified in a way that reduces the loss to a defined minimum. The aim of the training is to let the machine learning model generate for each input data an output which comes as close to the corresponding target as possible. Once the defined minimum is reached, the (now fully trained) machine learning model can be used to predict an output for new input data (i.e., input data which have not been used during training and for which the target is usually not (yet) known).

In a preferred embodiment of the present disclosure, the machine learning model is or comprises an artificial neural network.

An "artificial neural network" (ANN) is a biologically inspired computational model. An ANN usually comprises at least three layers of processing elements: a first layer with input neurons (nodes), a *k^{th}* layer with at least one output neuron (node), and *k-2* inner layers, where *k* is a natural number greater than 2.

In such a network, the input neurons serve to receive the input data. If the input data constitute or comprise an n-dimensional vector (e.g., a feature vector), with *n* being an integer equal to or greater than 1, there is usually one input neuron for each component of the vector. The output neurons serve to output at least one value. In case of the machine learning model of the present disclosure, the ANN comprises two outputs, a first output and a second output. The reconstructed composition data are outputted via the first output, and the predicted property data are outputted via the second output. The processing elements of the layers are interconnected in a predetermined pattern with predetermined connection weights therebetween. Each network node represents a pre-defined calculation of the weighted sum of inputs from prior nodes and a non-linear output function. The combined calculation of the network nodes relates the inputs to the outputs. It should be noted that an ANN can also use connection biases b, i.e. the output *y* of a neuron given an input *x* is calculated as *y* = act(w *x* + *b*) where w denotes the weights and act denotes the activation function.

When trained, the connection weights between the processing elements in the ANN contain information regarding the relationship between the input data and the output data which can be used to predict new output data from new input data.

Training estimates network weights that allow the network to calculate output values close to the target values. The network weights can be initialized with small random values or with the weights of a prior partially trained network. The training data inputs are applied to the network and the output values are calculated for each training sample. The network output values are compared to the target values. A backpropagation algorithm can be applied to correct the weight values in directions that reduce the error between targeted and calculated outputs. The process is iterated until no further reduction in error can be made or until a predefined prediction accuracy has been reached.

A cross-validation method can be employed to split the data into training and validation data sets. The training data set is used in the backpropagation training of the network weights. The validation data set is used to verify that the trained network generalizes to make good predictions. The best network weight set can be taken as the one that best predicts the outputs of the training data. Similarly, varying the number of network hidden nodes and determining the network that performs best with the data sets optimizes the number of hidden nodes.

In a preferred embodiment of the present disclosure, the machine learning model comprises an encoder-decoder structure, also referred to as autoencoder.

An autoencoder is a type of artificial neural network used to learn efficient data encodings in an unsupervised manner. In general, the aim of an autoencoder is to learn a representation (encoding) for a set of data, typically for dimensionality reduction, by training the network to ignore "noise". Along with the reduction side (encoder), a reconstructing side (decoder) is learnt, where the autoencoder tries to generate from the reduced encoding a representation as close as possible to its original input (or a part thereof).

Fig. 2 shows schematically a preferred embodiment of the machine learning model of the present disclosure. The machine learning model comprises an encoder e, a decoder d, and a property prediction unit *p*.

The task of the encoder e is to generate a compressed representation CR of the composition data fed into the machine learning model. The task of the decoder d is to reconstruct at least a part of the composition data from the compressed representation CR. The reconstructed data rCD can be outputted. The property prediction unit *p* serves to predict property data, at least partially on the basis of the compressed representation CR. The predicted property data pPD can be outputted.

Further details about autoencoders and the generation of compressed representations can be found in various publications (see e.g., D. Bank et al.: Autoencoders, arXiv:2003.05991).

For the generation of composition data, the output of the artificial neural network may further be processed by explicitly defined heuristics derived from domain expertise on the development of formulations. For example, it might be the case that certain materials cannot be combined in one formulation. An explicitly defined heuristic could ensure that such constraints on formulation development are satisfied by the disclosed formulation development approach even if the composition data generated by the artificial neural network might violate these constraints.

The composition data used as input for the (trained and untrained) machine learning model are generated from composition information. Composition information provide information on the composition of a formulation. The following Table 1 shows an example.

**Table 1: Ingredients of a formulation**

| **Substance Name** | **Weight-%** | **Type** |
|---|---|---|
| Spirotetramat | 22.4 | active ingredient |
| Glycerine | 5 | auxiliary |
| 1,2-Benzisothiazol-3(2H)-one | 0.05 | active ingredient |
| Silicone Oil | 0.1 | auxiliary |
| Xanthan Gum | 0.5 | auxiliary |
| Water | 71.95 | auxiliary |

Table 1 lists the ingredients of a formulation in form of their substance names, as well as the amount of each ingredient in weight percent. In addition, for each ingredient a type is specified. The formulation of Table 1 contains two active ingredients. All other ingredients are referred to as auxiliaries in this example. It is possible to define further/other types, such as solvent, formulant, lubricant, surfactant, colorant, preservative, thickener, and/or others.

Composition information may comprise information about the type of formulation. Formulation types, for example, can broadly characterize the physical nature of formulations and, at best, implicitly convey some high-level guidelines for formulation preparation. However, formulation types can also be counted among formulation properties (property information, property data).

In a preferred embodiment of the present disclosure, composition information and/or property information comprise information about the type of the formulation according to FAO/WHO Manual Pesticide Specifications (https://www.fao.org/agriculture/crops/thematic-sitemap/theme/pests/jmps/ manual/en/).

Composition information may comprise further information about a formulation such as processing information about how to obtain the formulation from the ingredients. Such processing information may include information about which ingredients to mix together, in what fashion and in what order, how to mix the ingredients (e.g., stirring, shaking) under which conditions (e.g., temperature, pressure, stirring speed) and/or the like.

From composition information, composition data are generated. Composition data can be considered as a numerical representation of the composition information. Many algorithms in machine learning require a numerical representation of objects since such representations facilitate processing and statistical analysis. In this context, the term "feature vector" is often used in machine learning. The composition data may represent such a feature vector. However, the composition data are not necessarily limited to vectors; the composition data can also be matrices, tensors, or the like. This also applies analogously to the generation of property data from property information.

Preferably, composition data of a formulation comprise information about the type of at least some of the ingredients. Possible types are for example: active ingredient, solvent, formulant, lubricant, surfactant, colorant, preservative, thickener, and/or others.

Preferably, composition data comprise information about the chemical structure of at least some ingredients of a formulation, preferably on the molecular level.

Information about the chemical structure of an ingredient can be encoded, for example, by a structural representation of the compound, such as a SMILES, a SELFIES and/or the like.

A simplified molecular-input line-entry system (SMILES) is a structural representation of a chemical substance in the form of a line notation for describing the structure of the chemical substance using ASCII strings (see e.g., http://opensmiles.org/).

A self-referencing embedded string (SELFIES) is a string-based structural representation of a molecular graph, which has the advantage over a SMILES that each SELFIES corresponds to a valid molecule, even entirely random strings (see e.g.: M. Krenn et al.: Self-referencing embedded strings (SELFIES): A 100% robust molecular string representation, Mach. Learn.: Sci. Technol. 1 (2020) 045024, https://doi.org/10.1088/2632-2153/aba947).

However, other structural representations are also possible.

In a preferred embodiment, at least one active ingredient of a formulation is represented by a structural representation. In a preferred embodiment, each active ingredient of a formulation is represented by a structural representation.

Additionally or alternatively, composition data may comprise data about physical and/or chemical properties of ingredients.

For some ingredients, neither structural information nor information on physical and/or chemical properties may be available. Such ingredients may be represented by one-hot encodings.

One-hot encoding is a method to quantify categorical data. In short, this method produces a vector with length equal to the number of categories in the data set. If a data point belongs to the i^{th} category then components of this vector are assigned the value 0 except for the i^{th} component, which is assigned a value of 1.

A formulation can be composed of a defined number of ingredients, where the ingredients are selected from a defined number of available materials. If a number *N* of materials is available, then a number *n* can be selected from these *N* materials, which are then combined as ingredients of the formulation, wherein *N* and *n* are integers greater than or equal to 1, and *n* is equal to or smaller than *N.* The materials can be represented by an index i that can take the values from 1 to *N.* The composition of a formulation can be represented by a number of *n* vectors (a set of vectors), each vector representing one material which is an ingredient of the formulation. If the material with index i is used as an ingredient in the formulation, there is a vector for which the components of this vector are assigned the value 0 except for the i^{th} component, which is assigned a value of 1.

The amount (quantity) of each ingredient can be encoded by another dimension added to the set of vectors.

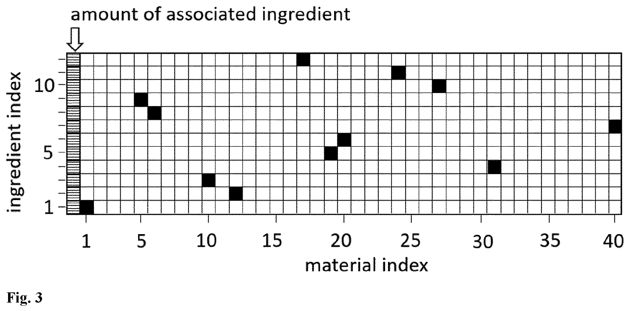

The representation of ingredients on the basis of one-hot encodings is schematically depicted in Fig. 3. In Fig. 3 the composition of the formulation is depicted as a two-dimensional array. On the horizontal axis, a material index is plotted. The material index can take the values from 1 to 40, so, there are 40 materials available. On the vertical axis an ingredient index is plotted. In case of the example depicted in Fig. 3, the ingredient axis can take the values from 1 to 12, so, the formulation of Fig. 3 contains 12 ingredients. The ingredient with the index 1 is the material with the index 1; the ingredient with the index 2 is the material with the index 12; and so on. Each horizontal line is a one-hot encoding of an ingredient of the formulation.

In Fig. 3, there is an additional dimension added to the array which encodes the amount of each ingredient in the formulation.

It is possible to add further dimensions, e.g., for specifying the type of each ingredient.

In a preferred embodiment, when generating the composition data from composition information, active ingredients are represented by structural representations and all other ingredients are represented by one-hot encodings. While new active ingredients are constantly being developed (e.g., in an effort to develop improved pesticides, pharmaceuticals or other products), new formulation aids (auxiliaries) are comparatively rare. In other words, when creating new formulations, a new active ingredient is often mixed with formulation aids selected from a number of known formulation aids. It is therefore possible to represent the active ingredients by a structural representation (which encodes structural information about the active ingredients), while the other ingredients (auxiliaries) are represented by one-hot encodings (which usually do not comprise any structural information about the other ingredients).

A vector representing an ingredient of a formulation, preferably including the amount of the ingredient, optionally including the type of the ingredient and/or further information related to the ingredient (e.g., lot number, production date, manufacturer and/or others) is also referred to as an element in this specification. In other words, the ingredients in a formulation are preferably represented by a set (in the mathematical sense) of elements, each element relating to an ingredient of the formulation.

The representation of the ingredients of formulations by a set of elements, as described herein, allows a permutation-invariant processing of formulations in machine learning models such as neural networks. In addition, the representation of ingredients of formulations by a set of elements as described herein, allows the processing of formulations having a variable number of ingredients. Both aspects are described in more detail below.

As already described, the machine learning model is *(inter alia)* trained to predict property data, at least partially on the basis of composition data.

During training, known (e.g., measured) property data are used as a target.

Property data are generated from information about one or more properties of formulations (i.e., property information). Preferably, the formulation properties are physical, chemical and/or biological properties. Examples of properties include but are not limited to physical and/or chemical stability, (wanted or unwanted) biological activity, bioavailability, (phyto)toxicity, viscosity, color, density, vapor pressure, decomposition temperature, freezing point, pH value and/or others. Property data can comprise numbers (such as viscosity or pH value) and/or categorial variables (such as stable/unstable). Property data can refer to defined conditions, such as measurement conditions (e.g., decomposition temperature for a defined pressure).

In a preferred embodiment of the present disclosure, the property data comprise data related to the stability of a formulation. Stability is usually a property that indicates whether a formulation remains in a form for a sufficiently long time to allow the formulation to be used for a defined purpose without significant degradation. Stability can be defined in different ways. For example, stability can be defined as a binary parameter (stable/unstable) that indicates whether the combination of ingredients results in a homogeneous mixture (stable) or whether inhomogeneities (for example a precipitation) occur (unstable). Stability can be defined as a binary parameter (stable/unstable) that indicates whether the combination of ingredients results in a formulation which under defined conditions (e.g., within a defined temperature and/or pressure range) retains one or more defined (other) properties (such as bioactivity, bioavailability, viscosity, and/or others). Stability can be defined as a quantity that indicates how long (shelf life) one or more properties of a formulation do not change (significantly, for example, without loss of quality) under given conditions (e.g., within a defined temperature and/or pressure range).

There are numerous tests for determining the stability of a formulation (see e.g., WHO Technical Report Series, No. 953, 2009, Annex 2: Stability testing of active pharmaceutical ingredients and finished pharmaceutical products; OECD Draft Guidance Document for Storage Stability Testing of Plant Protection and Biocidal Products, 6 January 2015). Property data related to the stability of a formulation can also be the result of such a stability test.

In a preferred embodiment of the present disclosure, stability is defined as the chemical stability of one or more active ingredients contained in a formulation. An active ingredient can decompose over time, where decomposition means that the active ingredient is transformed (e.g., by oxidation, hydrolytic cleavage and/or the like) into one or more other chemical compounds that have a different or lower (biological) activity than the original active ingredient. Stability can be measured, for example, as the proportion of the original amount (or concentration) of an active ingredient after a certain period of time under defined conditions (e.g., temperature, pressure, humidity, quantity of (sun) light, and/or the like). The higher the proportion, the higher the stability.

The property data can be a feature vector in which one or more properties of a formulation are represented by numbers. Examples for the generation of feature vectors can be found in the state of the art (see e.g., J. Frochte: Maschinelles Lernen, 2. Aufl., Hanser-Verlag 2019, ISBN: 978-3-446-45996-0).

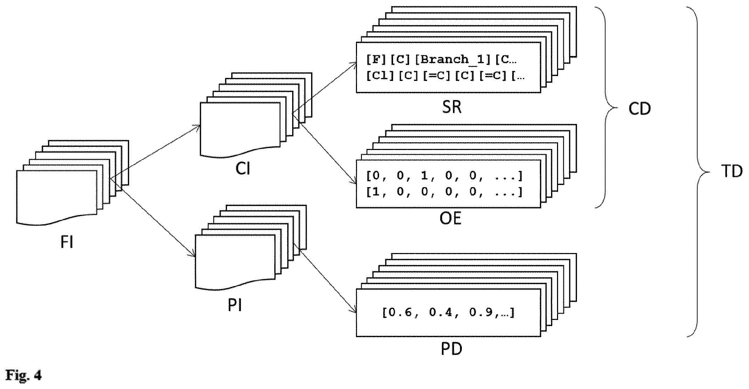

Fig. 4 shows schematically, by way of example, the process of generating training data on the basis of information about a multitude of formulations (formulation information).

The formulation information FI comprise, for each formulation of a number of formulations, composition information (CI) about the composition of the formulation and property information (PI) about one or more properties of the formulation. From composition information a set of structural representations SR is generated, each structural representation preferably representing one active ingredient of the formulation. Additionally, from the composition information a set of one-hot encodings OE is generated, each one-hot encoding preferably representing a further ingredient of the formulation.

A set of structural representations SR and the corresponding set of one-hot encodings OE constitute composition data CD for a formulation.

From the property information PI, property data PD are generated, the property data PD representing one or more properties of the formulation (in a machine-readable format, e.g., as a feature vector).

All composition data CD and property data PD form the training data TD.

During training, the composition data (and optionally further data) are fed into the machine learning model.

The machine learning model is configured to generate, at least partially on the basis of the composition data (and optionally further data), a compressed representation of the composition data (and optionally further data).

The machine learning model is further configured to perform two tasks, preferably simultaneously, on the basis of the compressed representation: a reconstruction task, and a prediction task. The reconstruction task aims at reconstructing some or all composition data from the compressed representation. The prediction task aims at predicting some or all property data from the compressed representation. The result of the reconstruction task is herein referred to as reconstructed composition data; the result of the prediction task is herein referred to as predicted property data.

A representation of a formulation in the feature space (e.g., in the form of composition data and property data) usually includes information that is relevant for performing the reconstruction task and the prediction task. However, it might also include other, irrelevant or redundant information. The "compressed representation" is a representation which contains or highlights only the relevant information with little or no influence from irrelevant and/or redundant information.

The compressed representation can, e.g., be generated by an encoder of an autoencoder as described above with respect to Fig. 2. The encoder compresses the input data into a latent space representation and the decoder attempts to recreate the input data from this latent space representation. The latent space representation is the compressed representation of the input data.

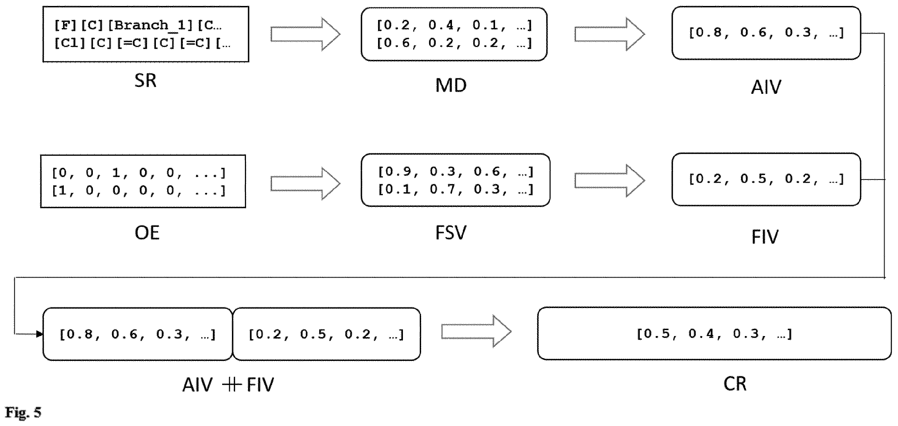

When looking at Fig. 4 again, it is apparent that for different formulations the number and sizes of the data sets (set of structural representations and set of one-hot encodings) can be different. The following describes how to input data sets, which may be of different sizes for different formulations, into the machine learning model to obtain a compressed representation. The description is based on Fig. 5.

Fig. 5 shows schematically, by way of example, a preferred approach to generate a compressed representation CR from a set of structural representations SR and a set of one-hot encodings OE.

In a first step, from each structural representation of the set of structural representations SR, a molecular descriptor MD can be generated. Preferably, the molecular descriptor is a numerical representation of fixed size of the respective active ingredient. Methods for generating such fixed-size molecular descriptors can be found in the scientific literature (see e.g.: R. Winter et al.: Learning continuous and data-driven molecular descriptors by translating equivalent chemical representations, Chem, Sci., 2019, 10, 1692ff). Preferably, a recurrent encoding is used to generate the molecular descriptors.

In a second step, from all the molecular descriptors, a joint representation can be generated, e.g., by pooling (e.g., by element-wise addition of the molecular descriptors, see e.g., M. Zaheer et al.: Deep Sets, arXiv:1703.06114v3). The joint representation is referred to herein as active ingredient(s) vector AIV.

In case of the set of one-hot-encodings OE, a transformer network can be used to generate a joint representation of fixed size of all further ingredients (see e.g.: A. Vaswani et al.: Attention is all you need, arXiv:1706.03762v5). Such a transformer network can account for pairwise interactions between elements of the input. The transformer framework can process sequential inputs while differentially weighting the contributions of each part of the input. By using several self-attention units, the transformer is capable of processing relevant interactions between two or more ingredients of the formulation. The output of the self-attention units is a set of vectors FSV which constitute numerical representations of the ingredients in the formulation while accounting for the presence of other ingredients in the formulation. A multi-head pooling unit can be used to compress this set of vectors FSV into a single vector to numerically represent all further ingredients of the formulation. The single vector representing all further ingredients of the formulation is referred to herein as further ingredients vector FIV. Multi-head pooling has been introduced by J. Lee et al. in Set Transformer: A Framework for Attention-based Permutation-Invariant Neural Networks, 2019, arXiv:1810.00825v3.

The active ingredient(s) vector AIV and the further ingredients vector FIV can be combined into the compressed representation CR, e.g., by a feedforward encoding.

The term "combination" as it is used herein means the combination of two or more pieces of data into a single piece of data. For example, two vectors may be combined to a single vector.

When two or more originally disjoint pieces of data are combined to one piece of data, they form a joint representation. Such a joint representation may comprise essentially, e.g., all or most of the information comprised in the individual pieces of data that the joint representation was derived of. The joint representation may also comprise a part of the information included in both or one of the individual pieces of data. Combination(s) of (e.g., at least two) representations to generate a joint representation may in case of vectors and/or matrices and/or tensors for example be achieved by a multiplication, an addition, for example an element-wise addition, a cross-product, concatenation (i.e., a stacking on top of each other), and many other procedures.

The process of generating a compressed representation as described herein enables permutation-invariant processing of formulations. Permutation-invariance means that the output of the machine learning model will not change under any permutation of the elements in the input set. For example, if a formulation comprising three components A, B, and C, the output of the model should be the same for each of the following representations R of the formulation: R={A, B, C}, R={A, C, B}, R={B, A, C}, R={B, C, A}, R={C, A, B}, R={C, B, A}.

Furthermore, the process of generating a compressed representation as described herein enables the processing of composition data of variable sizes. In the example depicted in Fig. 3, the formulation contains 12 ingredients. However, it is also possible that a formulation contains more than 12 ingredients or less than 12 ingredients. The machine learning model as described herein is able to handle formulations with any number (within a defined range) of ingredients.

The machine learning model is configured and trained to reconstruct, at least partially on the basis of the compressed representation, at least part of the composition data, e.g., by using a decoder as depicted in Fig. 2.

The decoder can reconstruct composition data, e.g., in a two-step process: First, the decoder draws independent and identically distributed samples from a high-dimensional probability distribution. In a preferred embodiment, this distribution is an isotropic normal distribution. As the samples are drawn independently every time the decoder generates composition data there is no inherent order to the randomly drawn samples. Then, the decoder conditions the samples on the latent representation generated by the encoder (i.e., vectors with entries [latent_dimension_0, latent_dimension_1, ..., latent_dimension_n, sample_dimension_0, sample_dimension_1, ..., sample_dimension_k] are constructed). Since all samples are conditioned on the same latent space representation, the decoder still does not impose a particular order to the samples. Finally, the decoder transforms the generated conditioned samples to the desired substances in several self-attention layers (which constitute permutation-equivariant operations) similar to the global pooling units in the encoder (but without the multi-head pooling).

Deviations between composition data and reconstructed composition data are preferably quantified using a loss function that does not require any specific ordering of the elements in the two sets that are compared, such as Hungarian loss, Hausdorff loss, or Chamfer loss. Suppose composition data represent a formulation F that is composed of ingredients A, B, and C such that F = {A, B, C}. The machine learning model reconstructs a formulation F' from the given formulation F with F' = {B', A', C'}. Preferably, the distance between F and F' is computed via the Hungarian loss or a similar loss function to account for the permutation invariance in the formulations. This approach would identify the permutation of the elements of F' which best matches F and compute the pairwise distance between the associated elements (A, A'), (B, B') and (C, C'). Preferably, distances between elements are calculated using a cross-entropy loss for the one-hot encodings and log-cosh loss for the amounts of each ingredients.

The machine learning model is further configured and trained to predict, at least partially on the basis of the compressed representation, property data, e.g., by using a property prediction unit as depicted in Fig. 2. Some examples for predicted properties are given below.

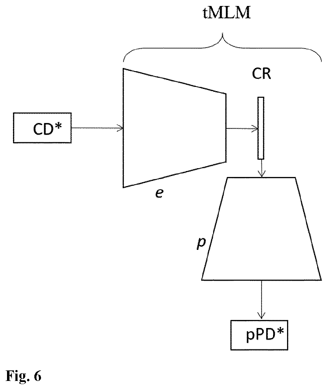

Once the machine learning model is trained, it can be used for prediction purposes. For example, it is possible to predict the properties of a new formulation. This is schematically shown in Fig. 6. New composition data CD* representing a new formulation are inputted into the trained machine learning model tMLM. The encoder e is configured to generate a compressed representation CR, at least partially on the basis of the new composition data CD*. The property prediction unit *p* is configured to generate predicted property data pPD* on the basis of the compressed representation CR. The predicted property data can be outputted (e.g., displayed on a monitor, printed on a printer and/or stored on a data storage). Note that for the prediction of property data, the decoder which was used for training the machine learning model (see e.g., Fig. 2) is not required.

If the predicted property data are promising (e.g., one or more properties are in a desired (pre-defined) range), the prediction results can be verified in a laboratory by producing the new formulation and measuring its properties.

In another use case, the aim is to find an optimized formulation for a given active ingredient, the optimized formulation having pre-defined desired properties.

Since the active ingredient is already pre-defined, the machine learning model is configured and trained to find, for the pre-defined active ingredient, those further formulation ingredients that will lead to a formulation with the desired properties (e.g., stability at a pre-defined viscosity).

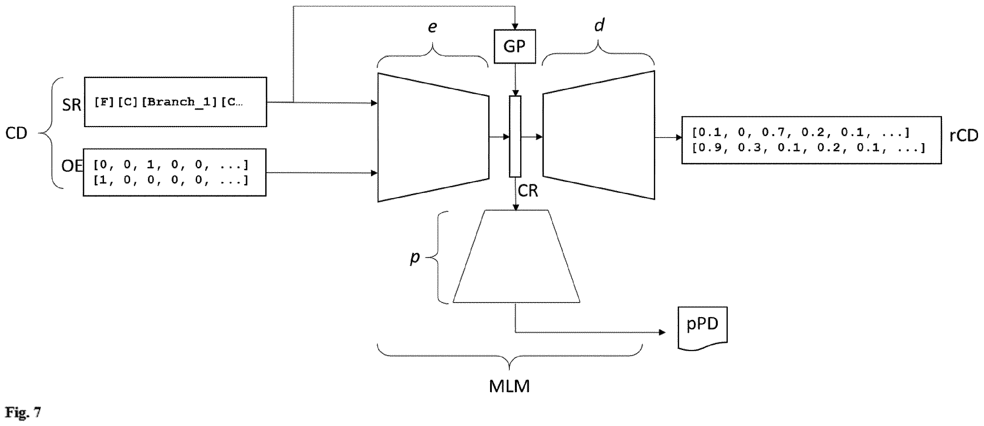

The training is therefore a variant of the training procedure described so far and is shown schematically in Fig. 7. The machine learning model MLM depicted in Fig. 7 comprises an encoder e, a decoder d, and a property prediction unit *p*. Composition data CD are used as input for the machine learning model MLM. The composition data CD comprise a structural representation SR representing the active ingredient of the formulation, and a set of one-hot encodings OE representing further ingredients of the formulation. In the example depicted in Fig. 7, there is only one active ingredient, however, it is also possible that there is more than one active ingredient. The structural representation SR and the set of one-hot encodings OE are inputted into the encoder e. The encoder e is configured and trained to generate a compressed representation CR at least partially on the basis of the inputted data. The structural representation SR is also fed into a global pooling unit GP. The global pooling unit GP is configured to generate a fixed size representation of the active ingredient which is entered into the generation of the compressed representation CR. The reason for this approach is that in the search for the optimized formulation, it is not the entire latent space that is of interest, but only the area associated with the given active ingredient.

The decoder d is configured to reconstruct only a part of the composition data from the compressed representation CR. The aim is to reconstruct data related to the further formulation ingredients, but not data related to the active ingredient.

The property prediction unit *p* is configured to predict property data pPD. The training can be limited to the desired property data (e.g., stability at a pre-defined viscosity).

The machine learning model can be trained using training data comprising a multitude of training data sets, the training data sets comprising composition data and property data for a multitude of active ingredient formulations. The active ingredient formulations can be formulations of the given active ingredient or formulations of the given active ingredient plus formulations of chemically similar active ingredients or formulations of chemically similar active ingredients.

"Chemically similar" means that two active ingredients have a pre-defined similarity. Two active ingredients can, e.g., be considered as being similar
- if the molecular structure of both active ingredients has one or more identical structural units, and/or
- if they belong to the same group of substances, and/or
- if they have the same mode of action, and/or
- if they are produced / consumed by the same or similar chemical / biological processes, and/or
- if one or more physical and/or chemical and/or biological properties are the same or lie in the same pre-defined range.

Similarity of two active ingredients can also be described as an inverse of a measure of distance in a descriptor space (see e.g., N. Nikolova, J. Jaworska: Approaches to Measure Chemical Similarity - a Review, https://doi.org/10.1002/qsar.200330831). Two active ingredients can be considered similar if their distance in a descriptor space is lower than a predefined threshold.

Once the machine learning model MLM depicted in Fig. 7 is trained, it can be used for prediction purposes.

It is possible to start the search for an optimized formulation with a known formulation with known properties. The known formulation can be, e.g., a formulation which has already a "promising" property profile. In a preferred embodiment of the present disclosure, the search starts with a known formulation that was used in the training process and that shows the best property profile of all formulations used in the training process. "Best" means that the properties of the known formulation are closest to the desired properties. However, it is also possible to select a formulation according to other criteria, or to select a formulation randomly.

Composition data related to the selected (e.g., known) formulation can be inputted into the trained machine learning model as described for the training process. The compressed representation CR generated by the encoder represents a point in latent space that is used as a starting point for exploration. From this starting point one can move in different directions of the latent space. Points are selected in the latent space which, starting from the starting point, are located in defined directions at a defined distance from the starting point. The points to be selected can, e.g., have the same distance to the starting point. Each selected point represents a formulation in the latent space. For each formulation represented by a selected point, the property prediction unit can be used to predict property data of the formulation. The coordinates of a selected point in the latent space correspond to the compressed representation of the respective formulation represented by the selected point. The coordinates can be inputted into the property prediction unit in order to predict the property data of the respective formulation. If the properties of the formulation represented by a selected point exceed the properties of the formulation represented by the starting point, the selected point can be used as a new starting point for searching formulations with further improved properties. Information about the composition of a newly found optimized formulation can be obtained by inputting its compressed representation into the decoder. The decoder will then output composition data for the newly found formulation.

The search can be stopped, e.g., if a formulation is found which meets pre-defined target criteria (target properties and/or preferred/desirable compositions). However, it is also possible to continue the search for other formulations which also satisfy the targeted properties but differ from the initially identified formulation. Collecting a reasonable diverse set of formulations, all of which are expected to satisfy desirable acceptance criteria, (i) increases the chance that at least one of the generated formulations actually satisfies the target criteria and (ii) provides some flexibility for laboratory personnel, who can draw inspiration from the generated set of formulations and enrich that set with their own ideas (or replace the generated formulations if they violate other constraints not taught to the machine learning model).

The process of exploring the latent space and identifying new formulations with improved properties can be automated with the help of a computer.

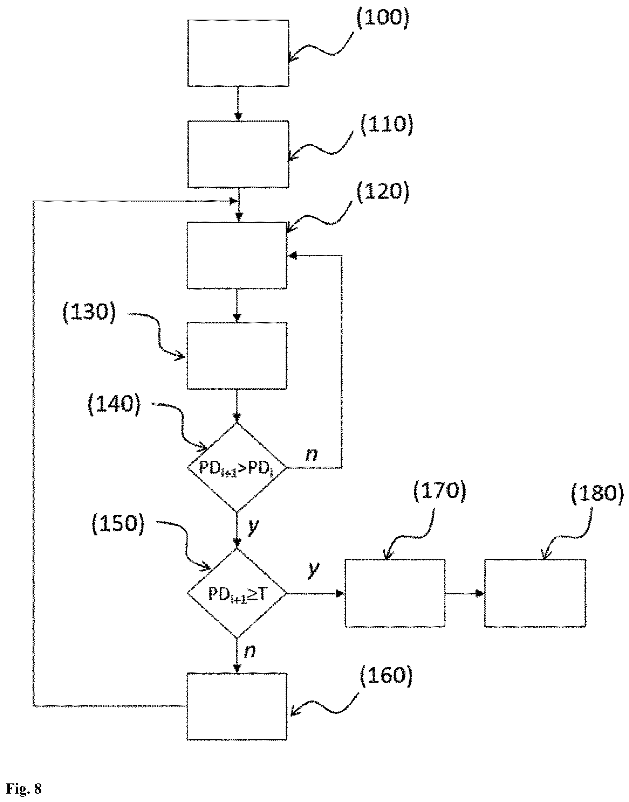

Fig. 8 shows an algorithm for the identification of optimized formulations.

In step (100), a starting point is determined. The starting point is a formulation i=0 characterized by composition data CD₀ and property data PD₀. In step (110), the composition data CD₀ are inputted into a trained machine learning model. The trained machine learning model comprises an encoder e, a decoder *d,* and a property prediction unit *p*. The encoder e is configured to generate, at least partially on the basis of the composition data CD₀, a compressed representation CR₀, the compressed representation CR₀ representing a point in latent space. In step (120), a point in the latent space is selected, the point to be selected having a pre-defined distance in a pre-defined direction from the point being represented by the compressed representation CR₀. The selected point corresponds to a compressed representation CR₁. In step (130), the compressed representation CR₁ is fed into the property prediction unit *p*. The property prediction unit is configured to compute (predict) property data PD₁ on the basis of the compressed representation CR₁. In step (140), the computed property data PD₁ are comparted with the property data PD₀ of the formulation that was used as a starting point. Comparison means that it will be determined whether the property data PD₁ are superior to the property data PD₀ (PD₁ > PD₀?). If the property data PD₁ are not superior to the property data PD₀ (PD₁ > PD₀ = *n),* the algorithm jumps back to step (120) and a new point in latent space is selected. If the property data PD₁ are superior to the property data PD₀ (PD₁ > PD₀= *y*), step (150) is initiated. In step (150), it is checked whether the property data PD₁ already meet pre-defined target criteria, e.g., whether the property data are PD₁ equal to or better than a predefined target (PD₁ ≥ T = ?). If the property data PD₁ are not equal to or not better than a pre-defined target (PD₁ ≥ T = *n),* step (160) is initiated. If the property data PD₁ are equal to or better than a predefined target (PD₁ ≥ T = *y),* step (170) is initiated. In step (160), the selected point in latent space corresponding to the compressed representation CR₁ is set as the new starting point for exploring the latent space (i→i+1), and the algorithm jumps back to step (120) in which a new point in latent space is selected that has a defined distance in a defined direction from the new starting point. In step (170), the compressed representation of the formulation which meets the pre-defined target criteria is fed into the decoder d in order to determine the composition data related to said formulation. In step (180), the composition data are transmitted to a laboratory in order to produce the formulation, and to measure real properties (e.g., in order to verify the predicted property data).

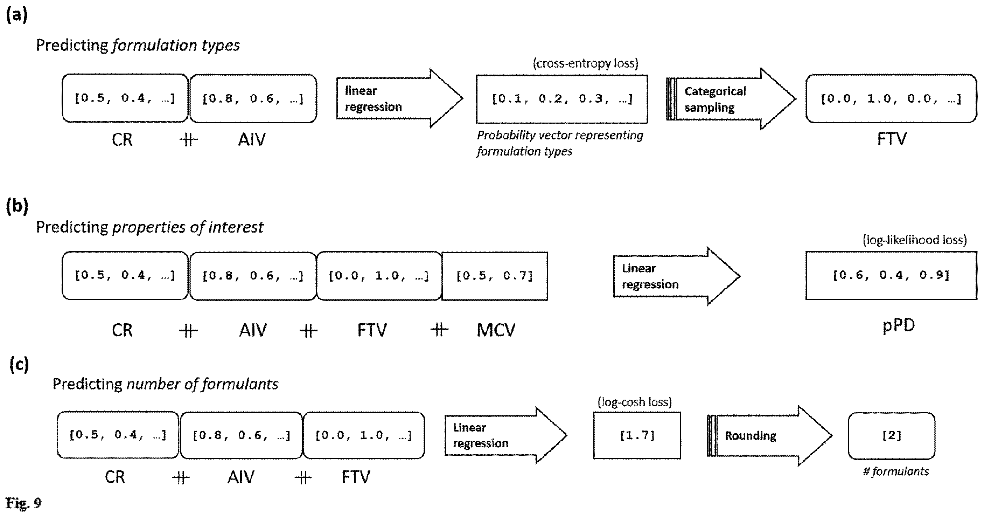

Below are further examples of how the trained machine learning model can be used for formulation development. The examples are described with reference to Fig. 9 (a) to Fig. 9 (f).

Fig. 9 (a) shows schematically, by way of example, a method of predicting a formulation type of a formulation. Formulations types can be treated as a formulation property. A formulation type can, e.g., be encoded as a one-hot-encoding. In the example shown in Fig. 9 (a) a compressed representation CR of a formulation is combined (e.g., concatenated) with an active ingredient vector AIV. Property data are predicted on the basis of this combined representation. The predicted property data represent a vector which indicates for all formulation types a probability that the present formulation is a formulation of the respective type. A categorial sampling reveals the one hot-encoded formulation type FTV.

Fig. 9 (b) shows schematically, by way of example, a method of predicting one or more properties of interest. In the example shown in Fig. 9 (a) the compressed representation CR is combined (e.g., concatenated) with the active ingredient vector AIV, the one-hot encoded formulation type FTV, and measurement conditions MCV under which the one or more properties of interest are usually measured. Property data are predicted on the basis of this combined representation. The predicted property data pPD are represented by a numerical vector.

Fig. 9 (c) shows schematically, by way of example, a method of predicting the number of further ingredients. In the example shown in Fig. 9 (c) the compressed representation CR is combined (e.g., concatenated) with the active ingredient vector AIV, and the one-hot encoded formulation type FTV. Property data are predicted on the basis of this combined representation. Rounding of the generated data reveals the number of formulants to be added to the active ingredient.

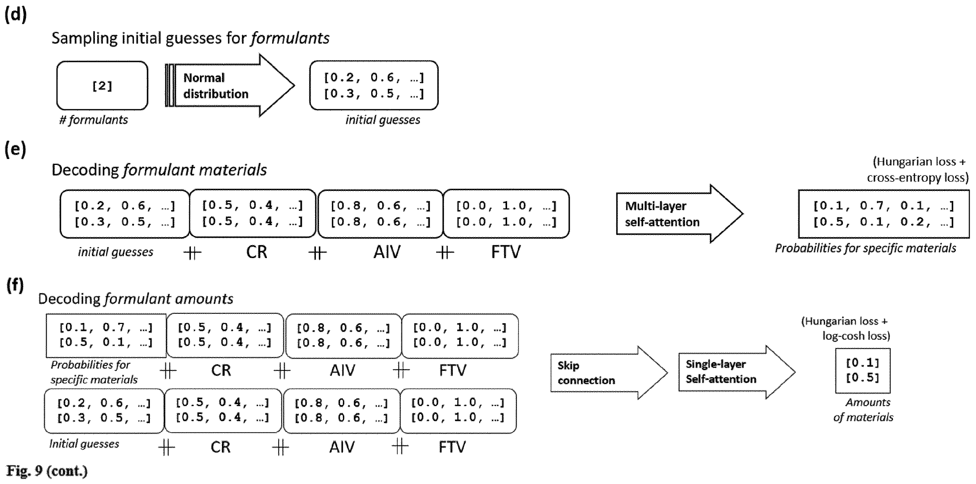

Fig. 9 (d) shows schematically, by way of example, how an initial guess for formulants (further ingredients) to be added to the active ingredient are generated.

Fig. 9 (e) shows schematically, by way of example, how the formulants materials can be decoded. The decoding is based on the vector representing the initial guess obtained as described for Fig. 9 (d) combined (e.g., concatenated) with the compressed representation CR, the active ingredient vector AIV, and the one-hot encoded formulation type FTV. A multi-layer self-attention reveals a set of vectors containing probabilities for the materials.

Fig. 9 (f) shows schematically, by way of example, how the amounts of each formulant can be predicted. Two vectors are used as input: a first vector constituting a combination (e.g., concatenation) of the set of vectors containing the probabilities for specific materials, the compressed representation CR, the active ingredient vector AIV, and the one-hot encoded formulation type FTV, and a second vector constituting a combination (e.g., concatenation) of the vector representing the initial guess, the compressed representation CR, the active ingredient vector AIV, and the one-hot encoded formulation type FTV. The decoding reveals the amounts of materials.

Any new and/or optimized formulation can be synthesized, and one or more predicted properties of the synthesized formulation can be experimentally verified, e.g., by performing one or more tests on or with the synthesized formulation.

Synthesizing a formulation and measuring its properties is not normally a step of the process of the invention, particularly since the process of the invention is a computer-implemented invention. However, it is possible that an examiner of a patent office may require that the process according to the invention have a direct effect on the real physical world beyond the usual interaction between hardware and software in running the computer-implemented process on a computer system.

Steps that may be part of the computer-implemented method may be steps to initiate synthesis of the formulation (e.g., sending a message to a synthesis laboratory as to which formulation to synthesize and/or automatically ordering chemicals/substances/ingredients to produce the formulation and/or steps to initiate measurement of one or more properties of the formulation (e.g., sending a message to a measurement laboratory as to which property to measure on which formulation).

Steps that may be performed following the computer-implemented method include synthesizing the formulation and/or performing a measurement of one or more properties on the formulation (e.g., to verify the predicted property(ies)).

The operations in accordance with the teachings herein may be performed by at least one computer system specially constructed for the desired purposes or at least one general-purpose computer system specially configured for the desired purpose by at least one computer program stored in a typically non-transitory computer readable storage medium.

The term "non-transitory" is used herein to exclude transitory, propagating signals or waves, but to otherwise include any volatile or non-volatile computer memory technology suitable to the application.

A "computer system" is a system for electronic data processing that processes data by means of programmable calculation rules. Such a system usually comprises a "computer", that unit which comprises a processor for carrying out logical operations, and also peripherals.

In computer technology, "peripherals" refer to all devices which are connected to the computer and serve for the control of the computer and/or as input and output devices. Examples thereof are monitor (screen), printer, scanner, mouse, keyboard, drives, camera, microphone, loudspeaker, etc. Internal ports and expansion cards are, too, considered to be peripherals in computer technology.

Computer systems of today are frequently divided into desktop PCs, portable PCs, laptops, notebooks, netbooks and tablet PCs and so-called handhelds (e.g., smartphone); all these systems can be utilized for carrying out the invention.

The term "process" as used above is intended to include any type of computation or manipulation or transformation of data represented as physical, e.g., electronic, phenomena which may occur or reside e.g. within registers and/or memories of at least one computer or processor. The term processor includes a single processing unit or a plurality of distributed or remote such units.

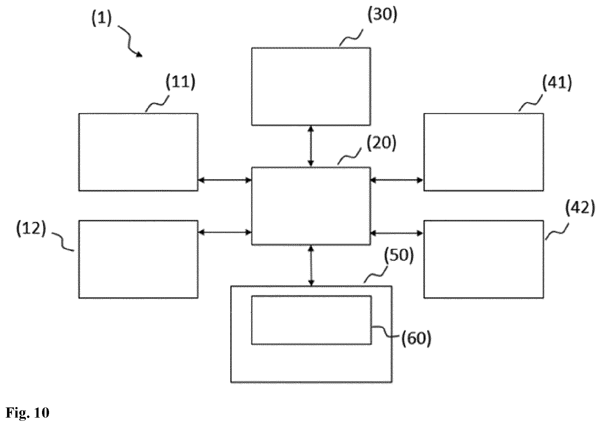

Fig. 10 illustrates a computer system (1) according to some example implementations of the present disclosure in more detail.

Generally, a computer system of exemplary implementations of the present disclosure may be referred to as a computer and may comprise, include, or be embodied in one or more fixed or portable electronic devices. The computer may include one or more of each of a number of components such as, for example, a processing unit (20) connected to a memory (50) (e.g., storage device).

The processing unit (20) may be composed of one or more processors alone or in combination with one or more memories. The processing unit (20) is generally any piece of computer hardware that is capable of processing information such as, for example, data, computer programs and/or other suitable electronic information. The processing unit (20) is composed of a collection of electronic circuits some of which may be packaged as an integrated circuit or multiple interconnected integrated circuits (an integrated circuit at times more commonly referred to as a "chip"). The processing unit (20) may be configured to execute computer programs, which may be stored onboard the processing unit (20) or otherwise stored in the memory (50) of the same or another computer.

The processing unit (20) may be a number of processors, a multi-core processor or some other type of processor, depending on the particular implementation. For example, it may be a central processing unit (CPU), a field programmable gate array (FPGA), a graphics processing unit (GPU) and/or a tensor processing unit (TPU). Further, the processing unit (20) may be implemented using a number of heterogeneous processor systems in which a main processor is present with one or more secondary processors on a single chip. As another illustrative example, the processing unit (20) may be a symmetric multi-processor system containing multiple processors of the same type. In yet another example, the processing unit (20) may be embodied as or otherwise include one or more ASICs, FPGAs or the like. Thus, although the processing unit (20) may be capable of executing a computer program to perform one or more functions, the processing unit (20) of various examples may be capable of performing one or more functions without the aid of a computer program. In either instance, the processing unit (20) may be appropriately programmed to perform functions or operations according to example implementations of the present disclosure.

The memory (50) is generally any piece of computer hardware that is capable of storing information such as, for example, data, computer programs (e.g., computer-readable program code (60)) and/or other suitable information either on a temporary basis and/or a permanent basis. The memory (50) may include volatile and/or non-volatile memory, and may be fixed or removable. Examples of suitable memory include random access memory (RAM), read-only memory (ROM), a hard drive, a flash memory, a thumb drive, a removable computer diskette, an optical disk, a magnetic tape or some combination of the above. Optical disks may include compact disk - read only memory (CD-ROM), compact disk - read/write (CD-R/W), DVD, Blu-ray disk or the like. In various instances, the memory may be referred to as a computer-readable storage medium. The computer-readable storage medium is a non-transitory device capable of storing information, and is distinguishable from computer-readable transmission media such as electronic transitory signals capable of carrying information from one location to another. Computer-readable medium as described herein may generally refer to a computer-readable storage medium or computer-readable transmission medium.

The training data, the machine learning model, and/or the trained machine learning model may be stored in the memory (50).

In addition to the memory (50), the processing unit (20) may also be connected to one or more interfaces for displaying, transmitting and/or receiving information. The interfaces may include one or more communications interfaces and/or one or more user interfaces. The communications interface(s) may be configured to transmit and/or receive information, such as to and/or from other computer(s), network(s), database(s) or the like. The communications interface may be configured to transmit and/or receive information by physical (wired) and/or wireless communications links. The communications interface(s) may include interface(s) (41) to connect to a network, such as using technologies such as cellular telephone, Wi-Fi, satellite, cable, digital subscriber line (DSL), fiber optics and the like. In some examples, the communications interface(s) may include one or more short-range communications interfaces (42) configured to connect devices using short-range communications technologies such as NFC, RFID, Bluetooth, Bluetooth LE, ZigBee, infrared (e.g., IrDA) or the like.

The user interfaces may include a display (30). The display may be configured to present or otherwise display information to a user, suitable examples of which include a liquid crystal display (LCD), light-emitting diode display (LED), plasma display panel (PDP) or the like. The user input interface(s) (11) may be wired or wireless, and may be configured to receive information from a user into the computer system (1), such as for processing, storage and/or display. Suitable examples of user input interfaces include a microphone, image or video capture device, keyboard or keypad, joystick, touch-sensitive surface (separate from or integrated into a touchscreen) or the like. In some examples, the user interfaces may include automatic identification and data capture (AIDC) technology (12) for machine-readable information. This may include barcode, radio frequency identification (RFID), magnetic stripes, optical character recognition (OCR), integrated circuit card (ICC), and the like. The user interfaces may further include one or more interfaces for communicating with peripherals such as printers and the like.

As indicated above, program code instructions (60) may be stored in memory (50), and executed by processing unit (20) that is thereby programmed, to implement functions of the systems, subsystems, tools and their respective elements described herein. As will be appreciated, any suitable program code instructions (60) may be loaded onto a computer or other programmable apparatus from a computer-readable storage medium to produce a particular machine, such that the particular machine becomes a means for implementing the functions specified herein. These program code instructions (60) may also be stored in a computer-readable storage medium that can direct a computer, processing unit or other programmable apparatus to function in a particular manner to thereby generate a particular machine or particular article of manufacture. The instructions stored in the computer-readable storage medium may produce an article of manufacture, where the article of manufacture becomes a means for implementing functions described herein. The program code instructions (60) may be retrieved from a computer-readable storage medium and loaded into a computer, processing unit or other programmable apparatus to configure the computer, processing unit or other programmable apparatus to execute operations to be performed on or by the computer, processing unit or other programmable apparatus.

Retrieval, loading and execution of the program code instructions (60) may be performed sequentially such that one instruction is retrieved, loaded and executed at a time. In some example implementations, retrieval, loading and/or execution may be performed in parallel such that multiple instructions are retrieved, loaded, and/or executed together. Execution of the program code instructions (60) may produce a computer-implemented process such that the instructions executed by the computer, processing circuitry or other programmable apparatus provide operations for implementing functions described herein.

Execution of instructions by processing unit, or storage of instructions in a computer-readable storage medium, supports combinations of operations for performing the specified functions. In this manner, a computer system (1) may include processing unit (20) and a computer-readable storage medium or memory (50) coupled to the processing circuitry, where the processing circuitry is configured to execute computer-readable program code instructions (60) stored in the memory (50). It will also be understood that one or more functions, and combinations of functions, may be implemented by special purpose hardware-based computer systems and/or processing circuitry which perform the specified functions, or combinations of special purpose hardware and program code instructions.

## Claims

1. A computer-implemented method comprising:
- receiving information about a multitude of formulations, the information comprising composition information about compositions of formulations, and property information about one or more properties of formulations,
- generating training data (TD) from the information about the multitude of formulations, the training data (TD) comprising composition data (CD), and property data (PD);
- providing a machine learning model (MLM);
- training the machine learning model (MLM), thereby generating a trained machine learning model (tMLM),
- storing the trained machine learning model (tMLM) and/or outputting the trained machine learning model (tMLM) and/or using the trained machine learning model (tMLM) for prediction purposes,
**characterized in that** the training comprises:
∘ inputting composition data (CD) into the machine learning model (MLM), wherein the machine learning model (MLM) is configured to
▪ generate a compressed representation (CR) of the formulation, at least partially on the basis of the composition data (CD),
▪ determine predicted property data (pPD), at least partially on the basis of the compressed representation (CR) of the formulation,
▪ generate reconstructed composition data (rCD), at least partially on the basis of the compressed representation (CR) of the formulation,
∘ computing a loss (L) by means of one or more loss function(s) (LF), the loss function(s) (LF) quantifying deviations between at least a part of the composition data (CD) and the reconstructed composition data (rCD), and between at least a part of the property data (PD) and the predicted property data (pPD),
o modifying parameters (MP) of the machine learning model (MLM) so that the loss (L) is minimized

2. The method according to claim 1, wherein each formulation is composed of one or more active ingredients and one or more further ingredients, wherein the composition data (CD) comprise a set of structural representations (SR), each structural representation representing an active ingredient, wherein the structural representation is a fixed-size numerical representation of the active ingredient.

3. The method according to claim 1 or 2, wherein each formulation is composed of one or more active ingredients and one or more further ingredients, wherein the composition data (CD) comprise a set of one-hot encodings (OE), the set of one-hot encoding representing the further ingredients.

4. The method according to any one of claims 1 to 3, further comprising:
- generating, for each formulation of the multitude of formulations, a set of structural representations (SR) from the composition information, each structural representation being a fixed-size numerical representation of the molecular structure of an active ingredient of the formulation,
- generating, for each formulation of the multitude of formulations, a set of one-hot encodings (OE) from the composition information, each one-hot encoding representing a further ingredient of the formulation,
- generating, for each formulation of the multitude of formulations, a permutation-invariant representation of all active ingredients contained in the formulation on the basis of the set of structural representations (SR),
- generating, for each formulation of the multitude of formulations, a permutation-invariant representation of all further ingredients contained in the formulation on the basis of the set of one-hot encodings (OE),
- generating, for each formulation of the multitude of formulations, a compressed representation (CR) of the formulation on the basis of the permutation-invariant representation of all active ingredients and the permutation-invariant representation of all further ingredients.

5. The method according to any one of claims 1 to 4, wherein deviations between composition data (CD) and reconstructed composition data (rCD) are quantified using a loss function (LF) that does not require a specific ordering of the elements in the two sets that are compared.

6. The method according to any one of claims 1 to 5, wherein the property data (PD) represent physical, chemical and/or biological properties of the formulations, in particular one or more properties selected from: physical and/or chemical stability, biological activity, bioavailability, (phyto)toxicity, viscosity, color, density, vapor pressure, decomposition temperature, freezing point, pH value.

7. The method according to any one of claims 1 to 6, wherein the machine learning model (MLM) comprises an encoder (e), a decoder (d), and a prediction unit (p), wherein the encoder (e) is configured and trained to generate, at least partially on the basis of the composition data (CD), a compressed representation (CR) of the formulation, wherein the decoder (*d*) is configured and trained to reconstruct, at least partially on the basis of the compressed representation (CR), at least a part of the composition data (CD), wherein the prediction unit (*p*) is configured and trained to predict, at least partially on the basis of the compressed representation (CR), property data (pPD).

8. The method according to any one of claims 1 to 7, wherein the formulations of the multitude of formulations are different formulations of chemically similar active ingredients, wherein two active ingredients are considered as being chemically similar
- if the molecular structure of both active ingredients has one or more identical structural units, and/or
- if they belong to the same group of substances, and/or
- if they have the same mode of action, and/or
- if they are produced / consumed by the same or similar chemical / biological processes, and/or
- if one or more physical and/or chemical and/or biological properties are the same or lie in the same pre-defined range, and/or
- structural descriptors of the active ingredients have a distance in descriptor space which is lower than a pre-defined threshold distance.

9. The method according to any one of claims 1 to 8, further comprising:
- predicting one or more properties of a new formulation, wherein the predicting comprises:
∘ selecting the new formulation,
∘ generating composition data (CD*) representing the composition of the new formulation,
∘ inputting the composition data (CD*) into the trained machine learning model (tMLM),
∘ receiving from the trained machine learning model (tMLM) predicted property data (pPD*),
∘ outputting the predicted property data (pPD*), and/or storing the predicted property data (pPD*) on a data storage.

10. The method according to any one of claims 1 to 8, further comprising:
- identification of one or more optimized formulations, wherein the identification comprises:
i) selecting an initial formulation,
ii) generating composition data (CD*) representing the composition of the initial formulation,
iii) generating a compressed representation (CR) of the initial formulation on the basis of the composition data (CD*), the compressed representation (CR) corresponding to a starting point in a latent space,
iv) selecting a point in latent space, the point to be selected having a pre-defined distance in a pre-defined direction from the starting point, the point to be selected representing another formulation,
v) determining one or more properties of the other formulation,
vi) comparing the determined properties of the other formulation with one or more target properties,
vii) in case the one or more determined properties are equal to or exceed the target properties: determining the composition of the other formulation and outputting the composition and the one or more properties,
viii) in case the one or more determined properties are not equal to and do not exceed the target properties: repeating steps iv) to viii) once or several times.

11. A method of claim 9 or 10, further comprising:
- initiating synthesis of the one or more new formations or the one or more optimized formulations and/or initiating measurement of one or more properties of the one or more new formulations or the one or more optimized formulations,
wherein initiating synthesis comprises: transmitting a message to a synthesis laboratory as to which formulation(s) to synthesize and/or ordering chemicals and/or substances and/or ingredients to produce the one or more new or optimized formulation,
wherein initiating measurement comprises: transmitting a message to a measurement laboratory as to which property/properties to measure on which formulation(s).

12. A computer system (1) comprising:
a processor (20); and
a memory (50) storing an application program (60) configured to perform, when executed by the processor, an operation, the operation comprising:
- receiving information about a multitude of formulations, the information comprising composition information about compositions of formulations, and property information about one or more properties of formulations,
- generating training data (TD) from the information about the multitude of formulations, the training data comprising composition data (CD), and property data (PD);
- providing a machine learning model (MLM);
- training the machine learning model (MLM), thereby generating a trained machine learning model (tMLM),
- storing the trained machine learning model (tMLM) and/or outputting the trained machine learning model (tMLM) and/or using the trained machine learning model (tMLM) for prediction purposes,
**characterized in that** the training comprises:
∘ inputting composition data (CD) into the machine learning model (MLM), wherein the machine learning model (MLM) is configured to
▪ generate a compressed representation (CR) of the formulation, at least partially on the basis of the composition data (CD),
▪ determine predicted property data (pPD), at least partially on the basis of the compressed representation (CR) of the formulation,
▪ generate reconstructed composition data (rCD), at least partially on the basis of the compressed representation (CR) of the formulation,
∘ computing a loss (L) by means of one or more loss function(s) (LF), the loss function(s) (LF) quantifying deviations between at least a part of the composition (CD) and the reconstructed composition data (rCD), and between at least a part of the property data (PD) and the predicted property data (pPD),
∘ modifying parameters (MP) of the machine learning model (MLM) so that the loss (L) is minimized.

13. A non-transitory computer-readable storage medium having stored thereon software instructions that, when executed by a processor (20) of a computer system (1), cause the computer system (1) to execute the following steps:
- receiving information about a multitude of formulations, the information comprising composition information about compositions of formulations, and property information about one or more properties of formulations,
- generating training data (TD) from the information about the multitude of formulations, the training data comprising composition data (CD), and property data (PD);
- providing a machine learning model (MLM);
- training the machine learning model (MLM), thereby generating a trained machine learning model (tMLM),
- storing the trained machine learning model (tMLM) and/or outputting the trained machine learning model (tMLM) and/or using the trained machine learning model (tMLM) for prediction purposes,
**characterized in that** the training comprises:
∘ inputting composition data (CD) into the machine learning model (MLM), wherein the machine learning model (MLM) is configured to
▪ generate a compressed representation (CR) of the formulation, at least partially on the basis of the composition data (CD),
▪ determine predicted property data (pPD), at least partially on the basis of the compressed representation (CR) of the formulation,
▪ generate reconstructed composition data (rCD), at least partially on the basis of the compressed representation (CR) of the formulation,
∘ computing a loss (L) by means of one or more loss function(s) (LF), the loss function(s) (LF) quantifying deviations between at least a part of the composition data (CD) and the reconstructed composition data (rCD), and between at least a part of the property data (PD) and the predicted property data (pPD),
∘ modifying parameters (MP) of the machine learning model (MLM) so that the loss (L) is minimized

## Patentansprüche

1. Computerimplementiertes Verfahren, umfassend:
- Empfangen von Informationen über eine Vielzahl von Formulierungen, wobei die Informationen Zusammensetzungsinformationen über Zusammensetzungen von Formulierungen und Eigenschaftsinformationen über eine oder mehrere Eigenschaften von Formulierungen umfassen,
- Erzeugen von Trainingsdaten (TD) aus den Informationen über die Vielzahl von Formulierungen, wobei die Trainingsdaten (TD) Zusammensetzungsdaten (CD) und Eigenschaftsdaten (PD) umfassen;
- Bereitstellen eines Modells für maschinelles Lernen (MLM) ;
- Trainieren des Modells für maschinelles Lernen (MLM), wodurch ein trainiertes Modell für maschinelles Lernen (tMLM) erzeugt wird,
- Speichern des trainierten Modells für maschinelles Lernen (tMLM) und/oder Ausgeben des trainierten Modells für maschinelles Lernen (tMLM) und/oder Verwenden des trainierten Modells für maschinelles Lernen (tMLM) zu Vorhersagezwecken,
**dadurch gekennzeichnet, dass** das Trainieren Folgendes umfasst:
∘ Eingeben von Zusammensetzungsdaten (CD) in das Modell für maschinelles Lernen (MLM), wobei das Modell für maschinelles Lernen (MLM) zu Folgendem ausgelegt ist:
▪ Erzeugen einer komprimierten Darstellung (CR) der Formulierung zumindest teilweise basierend auf den Zusammensetzungsdaten (CD),
▪ Bestimmen von vorhergesagten Eigenschaftsdaten (pPD) zumindest teilweise basierend auf der komprimierten Darstellung (CR) der Formulierung,
▪ Erzeugen von rekonstruierten Zusammensetzungsdaten (rCD) zumindest teilweise basierend auf der komprimierten Darstellung (CR) der Formulierung,
∘ Berechnen eines Verlusts (L) mittels einer oder mehrerer Verlustfunktionen (LF), wobei die Verlustfunktion(en) (LF) Abweichungen zwischen mindestens einem Teil der Zusammensetzungsdaten (CD) und den rekonstruierten Zusammensetzungsdaten (rCD) und zwischen mindestens einem Teil der Eigenschaftsdaten (PD) und den vorhergesagten Eigenschaftsdaten (pPD) quantifiziert/quantifizieren,
∘ Modifizieren von Parametern (MP) des Modells für maschinelles Lernen (MLM), so dass der Verlust (L) minimiert wird.

2. Verfahren nach Anspruch 1, wobei jede Formulierung aus einem oder mehreren Wirkstoffen und einem oder mehreren weiteren Inhaltsstoffen zusammengesetzt ist, wobei die Zusammensetzungsdaten (CD) einen Satz von Strukturdarstellungen (SR) umfassen, wobei jede Strukturdarstellung einen Wirkstoff darstellt, wobei die Strukturdarstellung eine numerische Darstellung des Wirkstoffs mit fester Größe ist.

3. Verfahren nach Anspruch 1 oder 2, wobei jede Formulierung aus einem oder mehreren Wirkstoffen und einem oder mehreren weiteren Inhaltsstoffen zusammengesetzt ist, wobei die Zusammensetzungsdaten (CD) einen Satz von One-Hot-Codierungen (OE) umfassen, wobei der Satz von One-Hot-Codierungen die weiteren Inhaltsstoffe darstellt.

4. Verfahren nach einem der Ansprüche 1 bis 3, ferner umfassend:
- Erzeugen eines Satzes von Strukturdarstellungen (SR) aus den Zusammensetzungsinformationen für jede Formulierung der Vielzahl von Formulierungen, wobei jede Strukturdarstellung eine numerische Darstellung der Molekülstruktur eines Wirkstoffs der Formulierung mit fester Größe ist,
- Erzeugen eines Satzes von One-Hot-Codierungen (OE) aus den Zusammensetzungsinformationen für jede Formulierung der Vielzahl von Formulierungen, wobei jede One-Hot-Codierung einen weiteren Inhaltsstoff der Formulierung darstellt,
- Erzeugen einer permutationsinvarianten Darstellung aller in der Formulierung enthaltenen Wirkstoffe für jede Formulierung der Vielzahl von Formulierungen basierend auf dem Satz von Strukturdarstellungen (SR),
- Erzeugen einer permutationsinvarianten Darstellung aller in der Formulierung enthaltenen weiteren Inhaltsstoffe für jede Formulierung der Vielzahl von Formulierungen basierend auf dem Satz von One-Hot-Codierungen (OE),
- Erzeugen einer komprimierten Darstellung (CR) der Formulierung für jede Formulierung der Vielzahl von Formulierungen basierend auf der permutationsinvarianten Darstellung aller Wirkstoffe und der permutationsinvarianten Darstellung aller weiteren Inhaltsstoffe.

5. Verfahren nach einem der Ansprüche 1 bis 4, wobei Abweichungen zwischen Zusammensetzungsdaten (CD) und rekonstruierten Zusammensetzungsdaten (rCD) unter Verwendung einer Verlustfunktion (LF) quantifiziert werden, die keine spezifische Reihenfolge der Elemente in den zwei Sätzen erfordert, die verglichen werden.

6. Verfahren nach einem der Ansprüche 1 bis 5, wobei die Eigenschaftsdaten (PD) physikalische, chemische und/oder biologische Eigenschaften der Formulierungen darstellen, insbesondere eine oder mehrere Eigenschaften, die aus Folgenden ausgewählt werden: physikalische und/oder chemische Stabilität, biologische Aktivität, Bioverfügbarkeit, (Phyto-) Toxizität, Viskosität, Farbe, Dichte, Dampfdruck, Zersetzungstemperatur, Gefrierpunkt, pH-Wert.

7. Verfahren nach einem der Ansprüche 1 bis 6, wobei das Modell für maschinelles Lernen (MLM) einen Codierer (e), einen Decodierer (d) und eine Vorhersageeinheit (p) umfasst, wobei der Codierer (e) dazu ausgelegt und trainiert ist, eine komprimierte Darstellung (CR) der Formulierung zumindest teilweise basierend auf den Zusammensetzungsdaten (CD) zu erzeugen, wobei der Decodierer (d) dazu ausgelegt und trainiert ist, mindestens einen Teil der Zusammensetzungsdaten (CD) zumindest teilweise basierend auf der komprimierten Darstellung (CR) zu rekonstruieren, wobei die Vorhersageeinheit (*p*) dazu ausgelegt und trainiert ist, Eigenschaftsdaten (pPD) zumindest teilweise basierend auf der komprimierten Darstellung (CR) vorherzusagen.

8. Verfahren nach einem der Ansprüche 1 bis 7, wobei die Formulierungen der Vielzahl von Formulierungen unterschiedliche Formulierungen chemisch ähnlicher Wirkstoffe sind, wobei zwei Wirkstoffe als chemisch ähnlich betrachtet werden,
- falls die Molekülstruktur beider Wirkstoffe eine oder mehrere identische Struktureinheiten aufweist, und/oder
- falls sie derselben Gruppe von Substanzen angehören, und/oder
- falls sie den gleichen Wirkmechanismus aufweisen, und/oder
- falls sie durch gleiche oder ähnliche chemische/biologische Prozesse erzeugt/verbraucht werden, und/oder
- falls eine oder mehrere physikalische und/oder chemische und/oder biologische Eigenschaften gleich sind oder im gleichen vordefinierten Bereich liegen, und/oder
- Strukturdeskriptoren der Wirkstoffe einen Abstand im Deskriptor-Raum aufweisen, der kleiner als ein vordefinierter Schwellenabstand ist.

9. Verfahren nach einem der Ansprüche 1 bis 8, ferner umfassend:
- Vorhersagen einer oder mehrerer Eigenschaften einer neuen Formulierung, wobei das Vorhersagen Folgendes umfasst:
∘ Auswählen der neuen Formulierung,
∘ Erzeugen von Zusammensetzungsdaten (CD*), die die Zusammensetzung der neuen Formulierung darstellen,
∘ Eingeben der Zusammensetzungsdaten (CD*) in das trainierte Modell für maschinelles Lernen (tMLM),
∘ Empfangen von vorhergesagten Eigenschaftsdaten (pPD*) von dem trainierten Modell für maschinelles Lernen (tMLM),
∘ Ausgeben der vorhergesagten Eigenschaftsdaten (pPD*) und/oder Speichern der vorhergesagten Eigenschaftsdaten (pPD*) auf einem Datenspeicher.

10. Verfahren nach einem der Ansprüche 1 bis 8, ferner umfassend:
- Identifizieren einer oder mehrerer optimierter Formulierungen, wobei das Identifizieren Folgendes umfasst:
i) Auswählen einer Ausgangsformulierung,
ii) Erzeugen von Zusammensetzungsdaten (CD*), die die Zusammensetzung der Ausgangsformulierung darstellen,
iii) Erzeugen einer komprimierten Darstellung (CR) der Ausgangsformulierung basierend auf den Zusammensetzungsdaten (CD*), wobei die komprimierte Darstellung (CR) einem Startpunkt in einem latenten Raum entspricht,
iv) Auswählen eines Punkts in dem latenten Raum, wobei der auszuwählende Punkt einen vordefinierten Abstand in einer vordefinierten Richtung von dem Startpunkt aufweist, wobei der auszuwählende Punkt eine andere Formulierung darstellt,
v) Bestimmen einer oder mehrerer Eigenschaften der anderen Formulierung,
vi) Vergleichen der bestimmten Eigenschaften der anderen Formulierung mit einer oder mehreren Zieleigenschaften,
vii) für den Fall, dass die eine oder die mehreren bestimmten Eigenschaften gleich den Zieleigenschaften sind oder diese überschreiten: Bestimmen der Zusammensetzung der anderen Formulierung und Ausgeben der Zusammensetzung und der einen oder mehreren Eigenschaften,
viii) für den Fall, dass die eine oder die mehreren bestimmten Eigenschaften nicht gleich den Zieleigenschaften sind und diese nicht überschreiten: ein- oder mehrmaliges Wiederholen der Schritte iv) bis viii) .

11. Verfahren nach Anspruch 9 oder 10, ferner umfassend:
- Initiieren der Synthese der einen oder der mehreren neuen Formulierungen oder der einen oder der mehreren optimierten Formulierungen und/oder Initiieren der Messung einer oder mehrerer Eigenschaften der einen oder der mehreren neuen Formulierungen oder der einen oder der mehreren optimierten Formulierungen,
wobei das Initiieren der Synthese Folgendes umfasst: Senden einer Nachricht an ein Syntheselabor im Hinblick darauf, welche Formulierung(en) synthetisiert werden sollen, und/oder Bestellen von Chemikalien und/oder Substanzen und/oder Inhaltsstoffen zur Herstellung der einen oder mehreren neuen oder optimierten Formulierung (en),
wobei das Initiieren der Messung Folgendes umfasst: Senden einer Nachricht an ein Messlabor im Hinblick darauf, welche Eigenschaft/Eigenschaften an welcher/welchen Formulierung(en) gemessen werden sollen.

12. Computersystem (1), umfassend:
einen Prozessor (20); und
einen Speicher (50), der ein Anwendungsprogramm (60) speichert, das dazu ausgelegt ist, bei Ausführung durch den Prozessor eine Operation auszuführen, wobei die Operation Folgendes umfasst:
- Empfangen von Informationen über eine Vielzahl von Formulierungen, wobei die Informationen Zusammensetzungsinformationen über Zusammensetzungen von Formulierungen und Eigenschaftsinformationen über eine oder mehrere Eigenschaften von Formulierungen umfassen,
- Erzeugen von Trainingsdaten (TD) aus den Informationen über die Vielzahl von Formulierungen, wobei die Trainingsdaten Zusammensetzungsdaten (CD) und Eigenschaftsdaten (PD) umfassen;
- Bereitstellen eines Modells für maschinelles Lernen (MLM) ;
- Trainieren des Modells für maschinelles Lernen (MLM), wodurch ein trainiertes Modell für maschinelles Lernen (tMLM) erzeugt wird,
- Speichern des trainierten Modells für maschinelles Lernen (tMLM) und/oder Ausgeben des trainierten Modells für maschinelles Lernen (tMLM) und/oder Verwenden des trainierten Modells für maschinelles Lernen (tMLM) zu Vorhersagezwecken,
**dadurch gekennzeichnet, dass** das Trainieren Folgendes umfasst:
∘Eingeben von Zusammensetzungsdaten (CD) in das Modell für maschinelles Lernen (MLM), wobei das Modell für maschinelles Lernen (MLM) zu Folgendem ausgelegt ist:
▪ Erzeugen einer komprimierten Darstellung (CR) der Formulierung zumindest teilweise basierend auf den Zusammensetzungsdaten (CD),
▪ Bestimmen von vorhergesagten Eigenschaftsdaten (pPD) zumindest teilweise basierend auf der komprimierten Darstellung (CR) der Formulierung,
▪ Erzeugen von rekonstruierten Zusammensetzungsdaten (rCD) zumindest teilweise basierend auf der komprimierten Darstellung (CR) der Formulierung,
∘Berechnen eines Verlusts (L) mittels einer oder mehrerer Verlustfunktionen (LF), wobei die Verlustfunktion(en) (LF) Abweichungen zwischen mindestens einem Teil der Zusammensetzungsdaten (CD) und den rekonstruierten Zusammensetzungsdaten (rCD) und zwischen mindestens einem Teil der Eigenschaftsdaten (PD) und den vorhergesagten Eigenschaftsdaten (pPD) quantifiziert/quantifizieren,
∘ Modifizieren von Parametern (MP) des Modells für maschinelles Lernen (MLM), so dass der Verlust (L) minimiert wird.

13. Nichtflüchtiges, computerlesbares Speichermedium mit darauf gespeicherten Softwareanweisungen, die bei Ausführung durch einen Prozessor (20) eines Computersystems (1) das Computersystem (1) veranlassen, die folgenden Schritte auszuführen:
- Empfangen von Informationen über eine Vielzahl von Formulierungen, wobei die Informationen Zusammensetzungsinformationen über Zusammensetzungen von Formulierungen und Eigenschaftsinformationen über eine oder mehrere Eigenschaften von Formulierungen umfassen,
- Erzeugen von Trainingsdaten (TD) aus den Informationen über die Vielzahl von Formulierungen, wobei die Trainingsdaten Zusammensetzungsdaten (CD) und Eigenschaftsdaten (PD) umfassen;
- Bereitstellen eines Modells für maschinelles Lernen (MLM) ;
- Trainieren des Modells für maschinelles Lernen (MLM), wodurch ein trainiertes Modell für maschinelles Lernen (tMLM) erzeugt wird,
- Speichern des trainierten Modells für maschinelles Lernen (tMLM) und/oder Ausgeben des trainierten Modells für maschinelles Lernen (tMLM) und/oder Verwenden des trainierten Modells für maschinelles Lernen (tMLM) zu Vorhersagezwecken,
**dadurch gekennzeichnet, dass** das Trainieren Folgendes umfasst:
∘ Eingeben von Zusammensetzungsdaten (CD) in das Modell für maschinelles Lernen (MLM), wobei das Modell für maschinelles Lernen (MLM) zu Folgendem ausgelegt ist:
▪ Erzeugen einer komprimierten Darstellung (CR) der Formulierung zumindest teilweise basierend auf den Zusammensetzungsdaten (CD),
▪ Bestimmen von vorhergesagten Eigenschaftsdaten (pPD) zumindest teilweise basierend auf der komprimierten Darstellung (CR) der Formulierung,
▪ Erzeugen von rekonstruierten Zusammensetzungsdaten (rCD) zumindest teilweise basierend auf der komprimierten Darstellung (CR) der Formulierung,
∘ Berechnen eines Verlusts (L) mittels einer oder mehrerer Verlustfunktionen (LF), wobei die Verlustfunktion(en) (LF) Abweichungen zwischen mindestens einem Teil der Zusammensetzungsdaten (CD) und den rekonstruierten Zusammensetzungsdaten (rCD) und zwischen mindestens einem Teil der Eigenschaftsdaten (PD) und den vorhergesagten Eigenschaftsdaten (pPD) quantifiziert/quantifizieren,
∘ Modifizieren von Parametern (MP) des Modells für maschinelles Lernen (MLM), so dass der Verlust (L) minimiert wird.

## Revendications

1. Procédé mis en œuvre par ordinateur comportant :
- la réception d'informations concernant une multitude de formulations, les informations comportant des informations de composition concernant des compositions de formulations, et des informations de propriétés concernant une ou plusieurs propriétés de formulations,
- la génération de données d'apprentissage (TD) à partir des informations concernant la multitude de formulations, les données d'apprentissage (TD) comportant des données de composition (CD), et des données de propriétés (PD) ;
- la mise en place d'un modèle d'apprentissage automatique (MLM) ;
- l'entraînement du modèle d'apprentissage automatique (MLM), générant ainsi un modèle d'apprentissage automatique entraîné (tMLM),
- le stockage du modèle d'apprentissage automatique entraîné (tMLM) et/ou la délivrance du modèle d'apprentissage automatique entraîné (tMLM) et/ou l'utilisation du modèle d'apprentissage automatique entraîné (tMLM) à des fins de prédiction,
**caractérisé en ce que** l'entraînement comporte :
∘ l'introduction de données de composition (CD) dans le modèle d'apprentissage automatique (MLM), le modèle d'apprentissage automatique (MLM) étant configuré pour
▪ générer une représentation compressée (CR) de la formulation, au moins partiellement d'après les données de composition (CD),
▪ déterminer des données de propriétés prédites (pPD), au moins partiellement d'après la représentation compressée (CR) de la formulation,
▪ générer des données de composition reconstituées (rCD), au moins partiellement d'après la représentation compressée (CR) de la formulation,
∘ le calcul d'une perte (L) au moyen d'une ou de plusieurs fonctions de perte (LF), la ou les fonctions de perte (LF) quantifiant des écarts entre au moins une partie des données de composition (CD) et les données de composition reconstituées (rCD), et entre au moins une partie des données de propriétés (PD) et les données de propriétés prédites (pPD),
∘ la modification de paramètres (MP) du modèle d'apprentissage automatique (MLM) de telle façon que la perte (L) soit minimisée.

2. Procédé selon la revendication 1, chaque formulation étant composée d'un ou de plusieurs ingrédients actifs et d'un ou de plusieurs ingrédients supplémentaires, les données de composition (CD) comportant un ensemble de représentations structurales (SR), chaque représentation structurale représentant un ingrédient actif, la représentation structurale étant une représentation numérique de taille fixée de l'ingrédient actif.

3. Procédé selon la revendication 1 ou 2, chaque formulation étant composée d'un ou de plusieurs ingrédients actifs et d'un ou de plusieurs ingrédients supplémentaires, les données de composition (CD) comportant un ensemble de codages disjonctifs complets (OE), l'ensemble de codages disjonctifs complets représentant les ingrédients supplémentaires.

4. Procédé selon l'une quelconque des revendications 1 à 3, comportant en outre :
- la génération, pour chaque formulation de la multitude de formulations, d'un ensemble de représentations structurales (SR) à partir des informations de composition, chaque représentation structurale étant une représentation numérique de taille fixée de la structure moléculaire d'un ingrédient actif de la formulation,
- la génération, pour chaque formulation de la multitude de formulations, d'un ensemble de codages disjonctifs complets (OE) à partir des informations de composition, chaque codage disjonctif complet représentant un ingrédient supplémentaire de la formulation,
- la génération, pour chaque formulation de la multitude de formulations, d'une représentation invariante vis-à-vis des permutations de tous les ingrédients actifs contenus dans la formulation d'après l'ensemble de représentations structurales (SR),
- la génération, pour chaque formulation de la multitude de formulations, d'une représentation invariante vis-à-vis des permutations de tous les ingrédients supplémentaires contenus dans la formulation d'après l'ensemble de codages disjonctifs complets (OE),
- la génération, pour chaque formulation de la multitude de formulations, d'une représentation compressée (CR) de la formulation d'après la représentation invariante vis-à-vis des permutations de tous les ingrédients actifs et la représentation invariante vis-à-vis des permutations de tous les ingrédients supplémentaires.

5. Procédé selon l'une quelconque des revendications 1 à 4, des écarts entre des données de composition (CD) et des données de composition reconstituées (rCD) étant quantifiés à l'aide d'une fonction de perte (LF) qui ne nécessite pas un ordonnancement particulier des éléments dans les deux ensembles qui sont comparés.

6. Procédé selon l'une quelconque des revendications 1 à 5, les données de propriétés (PD) représentant des propriétés physiques, chimiques et/ou biologiques des formulations, en particulier une ou plusieurs propriétés choisies parmi: la stabilité physique et/ou chimique, l'activité biologique, la biodisponibilité, la (phyto)toxicité, la viscosité, la couleur, la masse volumique, la pression de vapeur, la température de décomposition, le point de congélation, la valeur de pH.

7. Procédé selon l'une quelconque des revendications 1 à 6, le modèle d'apprentissage automatique (MLM) comportant un encodeur (e), un décodeur (d), et une unité de prédiction (*p*), l'encodeur (e) étant configuré et entraîné pour générer, au moins partiellement d'après les données de composition (CD), une représentation compressée (CR) de la formulation, le décodeur (d) étant configuré et entraîné pour reconstituer, au moins partiellement d'après la représentation compressée (CR), au moins une partie des données de composition (CD), l'unité de prédiction (p) étant configurée et entraînée pour prédire, au moins partiellement d'après la représentation compressée (CR), des données de propriétés (pPD).

8. Procédé selon l'une quelconque des revendications 1 à 7, les formulations de la multitude de formulations étant des formulations différentes d'ingrédients actifs chimiquement similaires, deux ingrédients actifs étant considérés comme étant chimiquement similaires
- si la structure moléculaire des deux ingrédients actifs comprend une ou plusieurs unités structurales identiques, et/ou
- s'ils appartiennent au même groupe de substances, et/ou
- s'ils ont le même mode d'action, et/ou
- s'ils sont produits / consommés par des processus chimiques / biologiques identiques ou similaires, et/ou
- si une ou plusieurs propriétés physiques et/ou chimiques et/ou biologiques sont les mêmes ou se situent dans la même plage prédéfinie, et/ou
- des descripteurs structuraux des ingrédients actifs présentent une distance dans un espace de descripteurs qui est inférieure à une distance seuil prédéfinie.

9. Procédé selon l'une quelconque des revendications 1 à 8, comportant en outre :
- la prédiction d'une ou de plusieurs propriétés d'une nouvelle formulation, la prédiction comportant :
∘ la sélection de la nouvelle formulation,
∘ la génération de données de composition (CD*) représentant la composition de la nouvelle formulation,
∘ l'introduction des données de composition (CD*) dans le modèle d'apprentissage automatique entraîné (tMLM),
∘ la réception, en provenance du modèle d'apprentissage automatique entraîné (tMLM), de données de propriétés prédites (pPD*),
∘ la délivrance des données de propriétés prédites (pPD*), et/ou le stockage des données de propriétés prédites (pPD*) sur un stockage de données.

10. Procédé selon l'une quelconque des revendications 1 à 8, comportant en outre :
- l'identification d'une ou de plusieurs formulations optimisées, l'identification comportant :
i) la sélection d'une formulation initiale,
ii) la génération de données de composition (CD*) représentant la composition de la formulation initiale,
iii) la génération d'une représentation compressée (CR) de la formulation initiale d'après les données de composition (CD*), la représentation compressée (CR) correspondant à un point de départ dans un espace latent,
iv) la sélection d'un point dans l'espace latent, le point à sélectionner présentant une distance prédéfinie dans une direction prédéfinie par rapport au point de départ, le point à sélectionner représentant une autre formulation,
v) la détermination d'une ou de plusieurs propriétés de l'autre formulation,
vi) la comparaison des propriétés déterminées de l'autre formulation avec une ou plusieurs propriétés cibles,
vii) dans le cas où la ou les propriétés déterminées égalent ou dépassent les propriétés cibles : la détermination de la composition de l'autre formulation et la délivrance de la composition et de la ou des propriétés,
viii) dans le cas où la ou les propriétés déterminées n'égalent pas et ne dépassent pas les propriétés cibles : la répétition des étapes iv) à viii) une ou plusieurs fois.

11. Procédé selon la revendication 9 ou 10, comportant en outre :
- l'amorce de la synthèse de la ou des nouvelles formulations ou de la ou des formulations optimisées et/ou l'amorce de la mesure d'une ou de plusieurs propriétés de la ou des nouvelles formulations ou de la ou des formulations optimisées,
l'amorce de la synthèse comportant : la transmission d'un message à un laboratoire de synthèse indiquant quelle(s) formulation(s) il convient de synthétiser et/ou passant commande de produits chimiques et/ou de substances et/ou d'ingrédients pour produire la ou les formulations nouvelles ou optimisées,
l'amorce de la mesure comportant : la transmission d'un message à un laboratoire de mesure indiquant quelle(s) propriété(s) il convient de mesurer sur quelle(s) formulation(s).

12. Système informatique (1) comportant :
un processeur (20) ; et
une mémoire (50) conservant un programme (60) d'application configuré pour effectuer, lorsqu'il est exécuté par le processeur, une opération, l'opération comportant :
- la réception d'informations concernant une multitude de formulations, les informations comportant des informations de composition concernant des compositions de formulations, et des informations de propriétés concernant une ou plusieurs propriétés de formulations,
- la génération de données d'apprentissage (TD) à partir des informations concernant la multitude de formulations, les données d'apprentissage comportant des données de composition (CD), et des données de propriétés (PD) ;
- la mise en place d'un modèle d'apprentissage automatique (MLM) ;
- l'entraînement du modèle d'apprentissage automatique (MLM), générant ainsi un modèle d'apprentissage automatique entraîné (tMLM),
- le stockage du modèle d'apprentissage automatique entraîné (tMLM) et/ou la délivrance du modèle d'apprentissage automatique entraîné (tMLM) et/ou l'utilisation du modèle d'apprentissage automatique entraîné (tMLM) à des fins de prédiction,
**caractérisé en ce que** l'entraînement comporte :
∘ l'introduction de données de composition (CD) dans le modèle d'apprentissage automatique (MLM), le modèle d'apprentissage automatique (MLM) étant configuré pour
▪ générer une représentation compressée (CR) de la formulation, au moins partiellement d'après les données de composition (CD),
▪ déterminer des données de propriétés prédites (pPD), au moins partiellement d'après la représentation compressée (CR) de la formulation,
▪ générer des données de composition reconstituées (rCD), au moins partiellement d'après la représentation compressée (CR) de la formulation,
∘ le calcul d'une perte (L) au moyen d'une ou de plusieurs fonctions de perte (LF), la ou les fonctions de perte (LF) quantifiant des écarts entre au moins une partie des données de composition (CD) et les données de composition reconstituées (rCD), et entre au moins une partie des données de propriétés (PD) et les données de propriétés prédites (pPD),
∘ la modification de paramètres (MP) du modèle d'apprentissage automatique (MLM) de telle façon que la perte (L) soit minimisée.

13. Support de stockage non transitoire lisible par ordinateur sur lequel sont stockées des instructions logicielles qui, lorsqu'elles sont exécutées par un processeur (20) d'un système informatique (1), amènent le système informatique (1) à exécuter les étapes suivantes :
- la réception d'informations concernant une multitude de formulations, les informations comportant des informations de composition concernant des compositions de formulations, et des informations de propriétés concernant une ou plusieurs propriétés de formulations,
- la génération de données d'apprentissage (TD) à partir des informations concernant la multitude de formulations, les données d'apprentissage comportant des données de composition (CD), et des données de propriétés (PD) ;
- la mise en place d'un modèle d'apprentissage automatique (MLM) ;
- l'entraînement du modèle d'apprentissage automatique (MLM), générant ainsi un modèle d'apprentissage automatique entraîné (tMLM),
- le stockage du modèle d'apprentissage automatique entraîné (tMLM) et/ou la délivrance du modèle d'apprentissage automatique entraîné (tMLM) et/ou l'utilisation du modèle d'apprentissage automatique entraîné (tMLM) à des fins de prédiction,
**caractérisé en ce que** l'entraînement comporte :
∘ l'introduction de données de composition (CD) dans le modèle d'apprentissage automatique (MLM), le modèle d'apprentissage automatique (MLM) étant configuré pour
▪ générer une représentation compressée (CR) de la formulation, au moins partiellement d'après les données de composition (CD),
▪ déterminer des données de propriétés prédites (pPD), au moins partiellement d'après la représentation compressée (CR) de la formulation,
▪ générer des données de composition reconstituées (rCD), au moins partiellement d'après la représentation compressée (CR) de la formulation,
∘ le calcul d'une perte (L) au moyen d'une ou de plusieurs fonctions de perte (LF), la ou les fonctions de perte (LF) quantifiant des écarts entre au moins une partie des données de composition (CD) et les données de composition reconstituées (rCD), et entre au moins une partie des données de propriétés (PD) et les données de propriétés prédites (pPD),
∘ la modification de paramètres (MP) du modèle d'apprentissage automatique (MLM) de telle façon que la perte (L) soit minimisée.
